(19) Europäisches Patentamt
European Patent Office
Office européen des brevets

(11) **EP 3 612 842 B1**

(12) **EUROPÄISCHE PATENTSCHRIFT**

(45) Veröffentlichungstag und Bekanntmachung des Hinweises auf die Patenterteilung:
**26.11.2025  Patentblatt 2025/48**

(21) Anmeldenummer: **18719534.2**

(22) Anmeldetag: **20.04.2018**

(51) Internationale Patentklassifikation (IPC):
*G01N 33/68* (2006.01)

(52) Gemeinsame Patentklassifikation (CPC):
**G01N 33/68; G01N 33/538;** G01N 2333/765

(86) Internationale Anmeldenummer:
**PCT/EP2018/060176**

(87) Internationale Veröffentlichungsnummer:
**WO 2018/193087 (25.10.2018 Gazette 2018/43)**

(54) **VERFAHREN ZUR BESTIMMUNG DER RELATIVEN BINDUNGSKAPAZITÄT DES ALBUMINS**

METHODS FOR DETERMINING THE RELATIVE BINDING CAPACITY OF ALBUMIN

PROCÉDÉ PERMETTANT DE DÉTERMINER LA CAPACITÉ DE LIAISON RELATIVE À L'ALBUMINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.04.2017  DE 102017206786**

(43) Veröffentlichungstag der Anmeldung:
**26.02.2020  Patentblatt 2020/09**

(73) Patentinhaber: **Fraunhofer-Gesellschaft zur Förderung**
**der angewandten Forschung e.V.**
**80686 München (DE)**

(72) Erfinder:
• **KLAMMT, Sebastian**
**10559 Berlin (DE)**
• **MITZNER, Steffen**
**18119 Rostock (DE)**
• **STANGE, Jan**
**18057 Rostock (DE)**
• **REISINGER, Emil**
**18147 Rostock (DE)**

(74) Vertreter: **Altmann Stößel Dick Patentanwälte PartG mbB**
**Theodor-Heuss-Anlage 2**
**68165 Mannheim (DE)**

(56) Entgegenhaltungen:
**WO-A2-02/23198**

• FRIEDMAN MENDEL: "Analysis, Nutrition, and Health Benefits of Tryptophan", INTERNATIONAL JOURNAL OF TRYPTOPHAN RESEARCH, vol. 11, 26 September 2018 (2018-09-26), pages 1 - 12, XP055882817
• WU ZHUONA ET AL: "Comparison of critical methods developed for fatty acid analysis: A review", JOURNAL OF SEPARATION SCIENCE, vol. 40, no. 1, 29 August 2016 (2016-08-29), DE, pages 288 - 298, XP055882823, ISSN: 1615-9306, Retrieved from the Internet <URL:https://api.wiley.com/onlinelibrary/tdm/v1/articles/10.1002%2Fjssc.201600707> DOI: 10.1002/jssc.201600707
• OETTL K ET AL: "Oxidative albumin damage in chronic liver failure: Relation to albumin binding capacity, liver dysfunction and survival", JOURNAL OF HEPATOLOGY, vol. 59, no. 5, 2013, pages 978 - 983, XP028755480, ISSN: 0168-8278, DOI: 10.1016/J.JHEP.2013.06.013
• ALTAMENTOVA S M ET AL: "A fluorescence method for estimation of toxemia: Binding capacity of lipoproteins and albumin in plasma", CLINICA CHIMICA ACTA, vol. 271, no. 2, 23 March 1998 (1998-03-23), pages 133 - 149, XP009118415, ISSN: 0009-8981

- **BRODERSEN R ET AL: "Determination of reserve albumin-equivalent for ligand binding, probing two distinct binding functions of the protein", ANALYTICAL BIOCHEMISTRY, vol. 121, no. 2, 1 April 1982 (1982-04-01), pages 395 - 408, XP024826642, ISSN: 0003-2697, [retrieved on 19820401], DOI: 10.1016/0003-2697(82)90499-7**
- **BLENCOWE T ET AL: "Benzodiazepine Whole Blood Concentrations in Cases With Positive Oral Fluid On-Site Screening Test Results Using the DrugWipe(R) Single for Benzodiazepines", JOURNAL OF ANALYTICAL TOXICOLOGY, vol. 35, 1 July 2011 (2011-07-01), pages 349 - 356, XP055479352, Retrieved from the Internet <URL:https://academic.oup.com/jat/article/35/6/349/770693> DOI: 10.1093/anatox/35.6.349**

**Beschreibung**

Die vorliegende Erfindung betrifft Verfahren zur Bestimmung der relativen Bindungskapazität des Albumins mittels Teststreifen. Insbesondere betrifft die Erfindung ein Verfahren zur Bestimmung der relativen Bindungskapazität des Albumins, welches die folgenden Schritte umfasst: a) Bereitstellen von mindestens zwei Messlösungen einer Test-Probe und einer Referenz-Probe, wobei die Messlösungen mindestens einen Albumin-bindenden Marker M enthalten und dieser mindestens eine Albumin-bindende Marker M in mindestens einer Messlösung der Test-Probe und der Referenz-Probe die vermutlich vorhandene Bindungskapazität des Albumins übersteigt und wobei die Test-Probe eine bestimmten Menge an Albumin mit unbekannter Bindungskapazität und die Referenz-Probe die gleiche bestimmte Menge an Albumin mit einer Referenz-Bindungskapazität aufweist; b) Inkubieren der Messlösungen unter Bedingungen, die es erlauben, dass der mindestens eine Albumin-bindende Marker M an Albumin binden kann, sodass Komplexe aus diesem Marker M und Albumin (M:A) entstehen; c) Abtrennen der in Schritt b) erzeugten Komplexe (M:A); d) Nachweisen des Vorhandenseins oder der Menge an ungebundenem Marker M in den Lösungen nach Abtrennen des Komplexes (M:A) durch mindestens einen Teststreifen, der die Bestimmung des ungebundenen Markers erlaubt; und e) Bestimmen der relativen Bindungskapazität des Albumins in der Test-Probe basierend auf dem Vorhandensein oder den nachgewiesenen Mengen an ungebundenem Marker M in Schritt d), wobei der mindestens eine Albumin-bindende Marker M ein Benzodiazepin, Tryptophan, eine Gallensäure, Dansylsarcosin, eine mittelkettige Fettsäure, Warfarin, Furosemid, ein Sulfonylharnstoff, Dansylamid, ein Opioid, Kokain oder ein Cannabinoid ist. Weiterhin betrifft die Erfindung ein Verfahren zur Bestimmung der Menge an funktionellem Albumin.

Albumin, das im menschlichen Plasma in der höchsten Konzentration vorkommende Eiweiß, ist neben anderen Funktionen, wie beispielsweise der Aufrechterhaltung des kolloidosmotischen Druckes, für den Transport von verschiedenen, meist lipophilen Substanzen im Organismus verantwortlich. Neben zahlreichen endogenen Substanzen, Stoffwechselprodukten und Hormonen, beispielsweise Fettsäuren, Bilirubin, Gallensäuren, Indoxylsulfat, Tryptophan, Steroide und Zytokine, wird auch eine große Anzahl von Medikamenten an Albumin gebunden im Organismus transportiert (Kragh-et al., Practical aspects of the ligand-binding and enzymatic properties of human serum albumin. Biol Pharm Bull 2002 Jun;25(6):695-704.)

Neben 7 Bindungsstellen für langkettigen Fettsäuren und einer freien SH-Gruppe am Cystein 34, beispielsweise für Stickstoffmonoxid, stehen zwei gruppenspezifische Bindungsstellen für die Vielzahl von körpereigenen (endogenen) oder applizierten (exogenen) Substanzen zur Verfügung. Bezug nehmend auf die Arbeiten zur Charakterisierung der Albuminbindungsstellen von Sudlow und Kollegen werden diese als Bindungsstellen I und II bezeichnet (Sudlow G, et.al., The characterization of two specific drug binding sites on human serum albumin. Mol Pharmacol 1975; 11(6): 824-832).Während an der Bindungsstelle I, oftmals auch als Warfarin/Bilirubin-Bindungsstelle bezeichnet, hauptsächlich heterozyklische Substanzen oder Dicarboxyl-Säuren gebunden werden, weisen die Liganden der Bindungsstelle II, oftmals auch als Diazepam/Indol-Bindungsstelle bezeichnet, hauptsächlich eine aromatische Grundstruktur auf.

Die Bindung einer Substanz an das Albuminmolekül kann durch Interaktionen mit anderen, die um die gleiche Bindungsstelle konkurrierenden albumingebundenen Substanzen, beeinflußt werden. Neben diesen kompetitiven Verdrängungsmechanismen können auch allosterische Wechselwirkungen oder posttranslationale strukturelle Veränderungen der Albuminstruktur, beispielsweise durch Carbamylation oder Glykation, die Albuminbindung von exogenen Toxinen und endogenen Substraten verändern (Lee P., Wu X., Review: modifications of human serum albumin and their binding effect. Curr Pharm Des 2015; 21(14):1862-1865 und Fassano M. et al., The extraordinary ligand binding properties of human serum albumin. IUBMB Life. 2005 Dec;57(12):787-96).

Unter physiologische Bedingungen ist der Albumin-Beladungszustand gering. Bei Vorliegen einer eingeschränkten Eliminationsfunktion, beispielsweise durch im Leber- und/oder Nierenversagen, kann es zu einer Ansammlung von lipophilen, albumingebundenen Substanzen im Blut und somit zu einer stärkeren Sättigung der Albuminbindungsstellen oder Überladung des Albuminmoleküls kommen. Dieses kann aufgrund von kompetitiven oder allosterischen Wechselwirkungen eine Erhöhung des wirksamen, ungebundenen Anteils von Substraten, Zytokinen, Hormonen und Arzneimitteln zur Folge haben und kann mit einer Beeinflussung von Stoffwechselregelkreisen und Veränderungen von pharmakologischen Wirkungen sowie stärkeren Nebenwirkungen vergesellschaftet sein.

Verschiedene Substanzen, die in erhöhten Konzentrationen im Plasma oder Liquor von Patienten im Leberversagen anzutreffen sind, konnten in den letzten Jahren identifiziert und ihre Bedeutung für den klinischen Verlauf des Leberversagens deutlicher gemacht werden. Seit längerem werden Ammoniak, kurz- und mittelkettige Fettsäuren, Mercaptane und Phenole als toxische Substanzen im Leberversagen angesehen. Anderen Stoffen wie Bilirubin, Gallensäuren oder bestimmten Aminosäuren wie Tryptophan, Phenylalanin oder Tyrosin wurde zumindest eine indirekte Beeinflussung des Stoffwechsels über Verdrängungsmechanismen am Albuminmolekül oder als Ausgangsstoffe für Phenole oder inhibitorische falsche Neurotransmitter zugeschrieben (Sen et al., Emerging indications for albumin dialysis. Am J Gastroenterol 2005 Feb;100(2):468-475).

Derzeit werden eine Anzahl verschiedener Toxine, Metaboliten oder Zytokine in einem direkten Zusammenhang mit den Komplikationen des akuten oder chronischen Leberversagens, wie Hepatische Enzephalopathie (HE), hyperdyname Zirkulation, portaler Hypertension, Cholestase, Pruritus, Aszites und hepatorenalem Syndrom (HRS) gesehen. Mit zunehmendem Erkenntnisstand über die pathogenetischen Beziehungen dieser Substanzen tritt auch die Frage ihrer therapeutischen Elimination und somit einer Beeinflussung der Schwere, des Verlaufes oder der Prognose des Leberversagens zunehmend in den Vordergrund (Hughes RD. Review of methods to remove protein-bound substances in liver failure. Int J Artif Organs 2002 Oct;25(10):911-917).

Durch eine Eliminierung albumingebundener Substanzen im Leberversagen mittels der Albumindialyse (meist in Form des Molecular Adsorbent Recirculating System - MARS®) konnten in randomisierten kontrollierten Studien Komplikationen des Leberversagens wie beispielsweise Nierenfunktion, mentale Funktion (Hepatische Enzephalopathie) oder die Hämodynamik vermieden bzw. gemindert und die Mortalität signifikant gesenkt werden (Heemann et al. Albumin dialysis in cirrhosis with superimposed acute liver injury: A prospective, controlled study. Hepatology 2002 Oct;36(4):949-958).

Im Nierenversagen kommt es aufgrund der unzureichenden Eliminierung zu einer Ansammlung von verschiedenen Urämietoxinen, darunter auch niedrigmolekulare albumingebundene Toxine. Indoxylsulfat und P-Cresol-Sulfat sind Prototypen dieser Urämietoxine und werden in klinischen und experimentellen Untersuchungen berücksichtigt. In klinischen Studien konnte gezeigt werden, dass die mit zunehmender Niereninsuffizienz steigenden Konzentrationen albumingebundener Urämietoxine toxische Wirkungen auf die Nieren und das Gefäßendothel aufweisen (Liabeuf et al., Protein-bound uremic toxins: new insight from clinical studies. Toxins (Basel) 2011 Jul;3(7):911-919). Indoxylsulfat hat pro-oxidative und pro-inflammatorische Eigenschaften und steigert die Expression von pro-inflammatorischen Zytokinen wie TGF-$\beta$. Weiterhin konnte eine toxinabhängige Fibrose des Nierengewebes durch Indoxylsulfat sowohl in einem 5/6-Nephrektomie- als auch in einem hypertensiven Ratten-Modell nachgewiesen werden. Pro-inflammatorische Effekte von P-Cresol-Sulfat auf Leukozyten ebenso wie ein Einfluss von Indoxylsulfat auf die Aortenkalzifikation und vaskuläre Gefäßsteifigkeit konnten ebenfalls dargestellt werden.

Eine direkte Wirkung von endogenen Urämietoxinen auf die Proliferation und Viabilität von Zellkulturen konnte bestätigt werden. Diese negative Beeinflussung ist wahrscheinlich funktioneller Natur, da sie nicht mit einer Steigerung der Apoptose einherging (Dou et al. The uremic solutes p-cresol and indoxyl sulfate inhibit endothelial proliferation and wound repair. Kidney Int 2004 Feb;65(2):442-451.) Ebenso konnte in diesen Experimenten ein negativer Einfluss albumingebundener Urämietoxine auf die Wundheilung dargestellt werden.

Unabhängig davon, ob die eingeschränkte Albuminfunktion im Organversagen durch eine erhöhte Beladung mit albumingebundenen Substanzen, allosterische Wechselwirkungen oder durch oxidative strukturelle Veränderungen des Albuminmoleküls oder durch eine Kombination dieser Faktoren bedingt ist, führt diese zu einer veränderten Bindung endogener Substanzen oder exogen zugeführter Medikamente und somit zu einer Zunahme des ungebundenen Anteils. Da jedoch nur der freie Anteil einer Substanz pharmakologisch wirksam ist, kommt es zu einer stärkeren Wirkung.

Ein hoher Beladungsgrad würde für erhöhte freie Konzentrationen von Albuminliganden und somit größere negative toxische Auswirkungen sprechen. Im Gegensatz dazu würden bei einer vorhandenen oder wiederhergestellten physiologischen Bindungsfunktion des Albuminmoleküls Wirkungen von Toxinen reduziert, der physiologische Plasmatransport von Substraten und Metaboliten aufrecht erhalten und die zu erwartenden pharmakokinetischpharmakodynamischen Beziehungen einer medikamentösen Therapie anzutreffen sein.

Eine Möglichkeit der Ermittlung der vorhandenen Bindungsfunktion des Albuminmoleküls stellt die Bestimmung und Summation der einzelnen Konzentration aller konkurrierenden Substanzen und die Abschätzung des molaren Beladungsgrades des Albuminmoleküls dar. Jedoch ist die Bestimmung von bestimmten Liganden, beispielsweise der Urämietoxinen Indoxylsulfat und P-Cresol-Sulfat, nur in einem aufwendigen technischen Verfahren mittels HPLC möglich. Auch wenn die Analytik dieser beiden Urämietoxine so weit vereinfacht werden könnte, dass eine rasche Analytik am Patientenbett möglich wäre, würde die fehlende Konzentrationsbestimmung der bekannten anderen und insbesondere der bisher noch unbekannten Toxine diesen Ansatz als nicht erfolgversprechend deklassieren.

Ein anderer Lösungsansatz wäre die Bestimmung des Albuminbeladungszustandes ohne Berücksichtigung der Art und Fraktionen der die Bindungsstelle besetzenden Substanzen. Wenn ein solcher einfach zu bestimmender Parameter zur Verfügung stehen würde, könnte dieser im Rahmen von klinischen Studien eingesetzt und die Eignung als prognostischer Parameter in Studien mit verschiedenen Patientenpopulationen überprüft werden. In Analogie, beispielsweise zur Bestimmung der Blutfette, wäre dann bei entsprechender Datenlage ein Einsatz in der Routinediagnostik zur Abschätzung der individuellen Prognose, die Auswahl der geeigneten patientenspezifischen Therapie sowie die Kontrolle des Therapieerfolges und Anpassung des Therapieplanes im Verlauf möglich.

Sowohl für das Leber- als auch für das Nierenversagen konnte eine Abhängigkeit der Albuminfunktion mit der Schwere der Erkrankung dargestellt werden (Klammt al. Albumin-binding function is reduced in patients with decompensated cirrhosis and correlates inversely with severity of liver disease assessed by model for end-stage liver disease. Eur J Gastroenterol Hepatol 2007 Mar;19(3):257-263.; Klammt et al. Albumin-binding capacity (ABiC) is reduced in patients with chronic kidney disease along with an accumulation of protein-bound uraemic toxins. Nephrol Dial Transplant 2011 Nov 15;27(6):2377-2383). In einer randomisierten kontrollierten klinischen Studie war die Elimination albumingebundener Substanzen mit einer Verbesserung der Albuminfunktion und der Mortalität vergesellschaftet. Patienten, bei denen in der ersten Therapiewoche eine Verbesserung der ABiC (Albumin Binding Capacity) zu verzeichnen hatten, wiesen ein signifikant höheres Überleben gegenüber Patienten ohne Verbesserung der Albuminfunktion auf (Klammt et al. Improvement of impaired albumin binding capacity in acute-on-chronic liver failure by albumin dialysis. Liver Transpl 2008 Aug 28;14(9):1333-1339). In einer ersten Pilotstudie konnte auch für die Gruppe der septischen Patienten eine eingeschränkte Albuminfunktion anhand des ABiC Tests gezeigt werden, die mit dem Schweregrad (SAPS II) korreliert (Hinz et al., Albumin function is reduced in severe sepsis. Infection 2011;39:S118).

EP 1 315 973 B1 offenbart ein indirektes Verfahren zur quantitativen Bestimmung der vorhandenenen Bindungskapazität des Albumins in einer wässrigen Lösung. In einer bevorzugten Ausführungsform wird die Markersubstanz Dansylsarkosin verwendet und dessen ungebundenen Anteil nach Bindung an ein definiertes Albumin als Fluoreszenzverstärker durch Fluoreszenzspektrophotometrie bestimmt.

Teststreifen zur Bestimmung von Substanzen wie Benzodiazepin sind beispielsweise aus Blencowe et al., (J. Analytical Tox, 35, 2011, Seiten 349-356) bekannt.

Für die fluorometrische Bestimmung der Bindungskapazität des Albumins sind jedoch verschiedene Arbeitsschritte notwendig, so dass die Bestimmung derzeit in einem zeitlichen Abstand zur Probenentnahme im Labor erfolgt. Wenn durch eine Vereinfachung der Analytik eine Bestimmung in der Nähe des Patienten (Point of care) möglich wäre, könnte die individuelle Therapieplanung, beispielsweise die Gabe von Infusionen, Dosierung von Medikamenten, Beginn, Dauer und Intensität extrakorporaler Verfahren, den gegenwärtigen Zustand des Patienten berücksichtigen und die Gefahr, einer mit Nebenwirkungen vergesellschaftete Übertherapie ebenso wie eine für den Patienten gefährliche Unterversorgung reduziert werden.

Als der vorliegenden Erfindung zugrunde liegende Aufgabe kann daher die Bereitstellung eines schnellen und einfachen Verfahrens zur Bestimmung der relativen Bindungskapazität des Albumins angesehen werden, welches eine Point-of-care Diagnostik ermöglicht.

Die Aufgabe wird durch die in den Ansprüchen und nachfolgend beschriebenen Ausführungsformen gelöst.

Die Erfindung betrifft somit ein Verfahren zur Bestimmung der relativen Bindungskapazität des Albumins umfassend:

a) Bereitstellen von mindestens zwei Messlösungen einer Test-Probe und einer Referenz-Probe, wobei die Messlösungen mindestens einen Albumin-bindenden Marker M enthalten und dieser mindestens eine Albumin-bindende Marker M in mindestens einer Messlösung der Test-Probe und der Referenz-Probe die vermutlich vorhandene Bindungskapazität des Albumins übersteigt und wobei die Test-Probe eine bestimmten Menge an Albumin mit unbekannter Bindungskapazität und die Referenz-Probe die gleiche bestimmte Menge an Albumin mit einer Referenz-Bindungskapazität aufweist;
b) Inkubieren der Messlösungen unter Bedingungen, die es erlauben, dass der mindestens eine Albumin-bindende Marker M an Albumin binden kann, sodass Komplexe aus diesem Marker M und Albumin (M:A) entstehen;
c) Abtrennen der in Schritt b) erzeugten Komplexe (M:A);
d) Nachweisen des Vorhandenseins oder der Menge an ungebundenem Marker M in den Lösungen nach Abtrennen des Komplexes (M:A) durch mindestens einen Teststreifen, der die Bestimmung des ungebundenen Markers erlaubt; und
e) Bestimmen der relativen Bindungskapazität des Albumins in der Test-Probe basierend auf dem Vorhandensein oder den nachgewiesenen Mengen an ungebundenem Marker M in Schritt d) wobei der mindestens eine Albumin-bindende Marker M ein Benzodiazepin, Tryptophan, eine Gallensäure, Dansylsarcosin, eine mittelkettige Fettsäure, Warfarin, Furosemid, ein Sulfonylharnstoff, Dansylamid, ein Opioid, Kokain oder ein Cannabinoid ist.

Das vorliegende Verfahren kann neben den zuvor genannten Schritten auch noch weitere Schritte umfassen. Ein weiterer Schritt kann beispielsweise die Zugabe von weiteren Stoffen sein, beispielsweise die Zugabe eines Stoffes zur Test- und Referenz-Probe zur Stabilisierung von Albumin.

Das Verfahren gemäß der vorliegenden Erfindung kann vorzugsweise automatisiert werden. Hierzu kann die Behandlung der Test- und/oder Referenz-Probe(n), beispielsweise das Inkubieren der Messlösungen und das Abtrennen der Komplexe aus diesem Marker M und Albumin (M:A)und/oder der Nachweis mittels Teststreifen und das Bestimmen der relativen Bindungskapazität des Albumins kann durch geeignete Robotik-Instrumente, Analyse-Roboter und/oder computerunterstützt durchgeführt werden.

Unter "Probe" im Sinne der vorliegenden Erfindung versteht man eine Lösung, die Albumin enthält. Bevorzugt handelt es sich um eine wässrige Lösung mit einem pH-Wert zwischen 5 und 8, besonders bevorzugt mit einem pH-Wert zwischen 7 und 8. Die sogenannte "Test-Probe" gemäß der Erfindung soll eine bestimmte Menge an Albumin mit unbekannter Bindungskapazität enthalten, während die sogenannte "Referenz-Probe" die gleiche bestimmten Menge an Albumin mit einer Referenz-Bindungskapazität enthalten soll. Was unter dem Begriff "Bindungskapazität" zu verstehen ist, wird an anderer Stelle hierin im Detail erläutert. Unter einer "bestimmten Menge" an Albumin versteht man eine ungefähr definierte Menge an Albumin, die messbar ist. Die Menge an Albumin wird gewöhnlich durch Ermittlung der Konzentration von Albumin in einer Lösung ermittelt. Methoden und Mittel zur Ermittlung der Konzentration von Albumin, beispielsweise der Menge von humanem Serumalbumin im Serum eines Patienten, sind dem Fachmann bekannt und beinhalten beispielsweise die Bestimmung mit Bromkresolgrün, Immunchemische Verfahren einschl. immunturbidimetrie und die (Eiweiß-)Elektrophorese. Es ist dem Fachmann zudem bekannt, dass sich typischerweise in einer Probe eines Menschen etwa zwischen 10 und 60 Gramm Albumin in einem Liter Blut nachweisen lassen bzw. menschliches Serum pro Liter bevorzugt etwa 35 bis 55 Gramm Albumin enthält. Es ist dem Fachmann darüber hinaus bekannt, dass die bestimmte Menge das Albumins Aussagen über das Vorhandensein der Anzahl an Albuminmolekülen zulässt, jedoch nicht über deren (Bindungs-) Funktion. Eine große bestimmte Menge an Albumin kann beispielsweise eine geringe Bindungsfunktionalität aufweisen, d.h. ein spezifisch an Albumin bindender Marker kann trotz Vorhandensein an Albuminmolekülen nicht mehr an Albumin binden, weil alle Bindungsstellen für den spezifisch an Albumin bindenden Marker an den vorhandenen Albuminmolekülen bereits belegt sind. Eine kleine bestimmte Menge an Albumin wiederum kann eine hohe Bindungsfunktionalität aufweisen, wenn beispielsweise bestimmte oder alle Bindungsstellen der Albuminmoleküle in der Lösung frei sind. Im Vergleich zur bestimmten Menge an Albumin, lässt somit die funktionelle Menge an Albumin bzw. die Menge an Albumin, die einen Albumin-bindenden Marker M aufnehmen kann, Rückschlüsse auf die Bindungskapazität des Albumins zu, was an anderer Stelle hierin im Detail erläutert wird.

Gemäß der Erfindung handelt es sich bei der "Test-Probe" bevorzugt um eine Blut-, Serum- oder Plasma-Probe eines Patienten. Bevorzugt als Testprobe ist weiterhin eine Albumin-haltige Lösung, i.e. eine wässrige Lösung, die Albumin enthält, besonders bevorzugt eine pharmazeutische Albuminpräparation oder Überstände aus Zellkulturen.

Als "Patient" wird im Allgemeinen ein menschliches Subjekt bezeichnet, welches von einem medizinischen Fachpersonal überwacht bzw. für einen medizinischen Zustand, eine Krankheit oder dergleichen behandelt wird. Bevorzugt handelt es sich bei dem Patienten im Sinne der Erfindung um ein menschliches Subjekt mit einer Leberschädigung und/oder eine Niereninsuffizienz und/oder Sepsis. Weiterhin bevorzugt ist ein Patient, der mehrere Albumingebundene Medikamente oder Substanzen erhalten soll oder erhalten hat, die an Albumin binden können. Die Begriffe Leberschädigung, Niereninsuffizienz und Sepsis umfassen hierbei sämtliche akute und chronische Krankheitszustände. Bevorzugt weist der Patient eine chronische Leberschädigung und/oder chronische Niereninsuffizienz und/oder eine schwere Sepsis mit sekundär bedingter Organschädigung auf. Symptome und Charakteristika der zuvor benannten Krankheiten sind dem Fachmann bekannt und werden beispielsweise in den Standardtextbüchern der Medizin, wie Stedman oder Pschyrembl, beschrieben. Patienten im Sinne der Erfindung können aber auch Tiere sein, z.B. Säugetiere und insbesondere Haus- und Nutztiere, wie Hunde, Katzen, Pferde, Kühe, Schweine oder Schafe, oder Labortiere, wie Mäuse oder Ratten.

Bei der "Referenz-Probe" handelt es sich gemäß der Erfindung bevorzugt um eine artifiziell hergestellte Albumin-Lösung oder eine Probe eines gesunden Subjekts der gleichen Spezies, im Folgenden auch "Proband" genannt. Der Begriff "artifiziell hergestellte Albumin-Lösung" umfasst jegliche Art einer wässrigen Lösung der Albumin zugesetzt wurde und/oder eine wässrige Lösung, von der bekannt ist, dass sie Albumin enthält. Eine artifiziell hergestellte Albumin-Lösung umfasst beispielsweise Albumin-haltige Lösungen wie pharmazeutische Albumin-Präparationen. Beispielsweise kann eine artifiziell hergestellte Albumin-Lösung eine Phosphatgepufferte Salzlösung (PBS-Lösung) sein, der eine bestimmte Menge an Albumin zugesetzt wurde. Weiterhin können pharmazeutisch hergestellte und/oder als Medizinprodukt kommerziell erhältlich Albumin-haltige Lösungen als Referenz-Lösung dienen. Gemäß der Erfindung ist die Referenz-Probe besonders bevorzugt eine Probe eines gesunden Probanden, eine gepoolte Probe von mehreren Probanden, eine pharmazeutische Albumin-haltige Präparation oder eine artifiziell hergestellte Albumin-Lösung.

Ein "gesunder Proband" ist bevorzugt ein Mensch, der dem Anschein nach gesund ist und von dem bekannt ist, dass er keine Leberschädigung, keine Niereninsuffizienz und keine Sepsis aufweist und somit keine stärkere Sättigung der Albuminbindungsstellen oder eine Überladung des Albuminmoleküls vorhanden ist. Die Probe eines gesunden Probanden im Sinne der Erfindung kann darüber hinaus eine "gepoolte" Probe sein, d.h. ein Gemisch mehrerer gesunder Probanden. Bevorzugt handelt es sich hierbei um gepoolte Plasma-Spenden mehrerer gesunder Probanden, die beispielsweise über Blutbanken verfügbar sein können. Die Referenz-Probe kann im Wesentlichen mit der Test-Probe übereinstimmen und sich lediglich durch die Bindungskapazität des Albumins unterscheiden, wie anderswo hierin im Detail erläutert wird.

Der Begriff "Messlösung" im Sinne der vorliegenden Erfindung bezeichnet eine Lösung, die eine Teilmenge der Test-Probe bzw. der Referenz-Probe umfasst und die mindestens einen Albumin-bindenden Marker M aufweist. Was unter dem Begriff "Albumin-bindender Marker M" zu verstehen ist, wird an anderer Stelle hierin erläutert. Eine Messlösung der Test-Probe kann beispielsweise ein Aliquot der Test-Probe oder eine verdünnte Lösung der Test-Probe sein, zu welcher der mindestens eine Albumin-bindenden Marker M gegeben wird. Gemäß der Erfindung sollen mindestens zwei Messlösungen der Test-Probe und der Referenz-Probe bereitgestellt werden, wobei der Albumin-bindende Marker M in mindestens einer Messlösung der Test-Probe und der Referenz-Probe die vermutlich vorhandene Bindungskapazität des Albumins (wie an anderer Stelle hierin erläutert) übersteigt. Vorzugsweise soll die Messlösung einen pH-Wert zwischen 5 und 8, besonders bevorzugt zwischen 7 und 8 aufweisen. Weiterhin kann die Messlösung zusätzliche Stoffe enthalten, beispielsweise Puffer und/oder Stabilisatoren, die der Stabilisierung des pH-Wertes und/oder der Stabilisierung von Albumin dienen. Dem Fachmann ist bekannt, dass im Serum eines gesunden Probanden die Konzentration von Albumin bei etwa 35 bis 55 Gramm pro Liter liegt und somit eine Teilmenge, beispielsweise eine Verdünnungslösung des Serums, eine Albumin-Konzentration von weniger als 55 Gramm pro Liter aufweisen dürfte. Dem Fachmann ist zudem bekannt, dass unterschiedliche Messlösungen, die verschiedene molare Verhältnisstufen von Marker M und Albumin aufweisen sollen, durch Verdünnungsreihen der Test-Probe und der Referenz-Probe bei Zugabe konstanter Mengen an Marker M oder durch Aliquotierung der Test-Probe und der Referenz-Probe bei Zugabe verschiedener Mengen an Marker M, erzeugt werden können. Bevorzugt sind beispielsweise molare Verhältnisstufen (Marker M/Albumin) von 1; 0,5; 0,3; 0,25; 0,2; 0,15; 0,1; und 0,05. Gemäß der Erfindung soll der Marker M in mindestens einer Messlösung der Test-Probe und der Referenz-Probe die vermutlich vorhandene Bindungskapazität des Albumins übersteigen, wie an anderer Stelle hierin erläutert wird.

Gemäß der Erfindung sollen mindestens zwei Messlösungen der Test-Probe und der Referenz-Probe bereitgestellt werden. Das Bereitstellen von mindestens zwei Messlösungen bedeutet, dass vorzugsweise zwei Messlösungen der Test-Probe und zwei Messlösungen der Referenz-Probe bereitgestellt werden. Schritt a) des erfindungsgemäßen Verfahrens umfasst somit bevorzugt das Bereitstellen von mindestens je zwei Messlösungen einer Test-Probe und einer Referenz-Probe. Bevorzugt ist weiterhin die Bereitstellung von mindestens 3, 4, 5, 6, 7 oder 8 Messlösungen der Test-Probe und der Referenz-Probe. Beispielsweise können die Test-Probe und die Referenz-Probe in jeweils 6 Aliquots aufgespalten werden, welche die gleiche bestimmte Menge an Albumin enthalten. Danach erfolgt die Zugabe unterschiedlicher Mengen eines Albumin-bindenden Markers M, sodass absteigende molare Verhältnisse von Marker zu Albumin in den jeweiligen Aliquots der Test-Probe und der Referenz-Probe entstehen. Alternativ können die Test-Probe und der Referenz-Probe absteigend verdünnt werden, beispielsweise können 6 Verdünnungslösungen der Test-Probe und der Referenz-Probe hergestellt werden, die jeweils unterschiedliche Mengen an Albumin enthalten. Danach erfolgt die Zugabe gleicher Mengen eines Albumin-bindenden Markers M, sodass wiederum verschiedene molare Verhältnisse von Marker zu Albumin in den jeweiligen 6 Verdünnungslösungen der Test-Probe und der Referenz-Probe vorliegen. Hierbei soll gemäß der Erfindung in mindestens einer Messlösung der Test-Probe und der Referenz-Probe der mindestens eine Albumin-bindende Marker M die vermutlich vorhandene Bindungskapazität des Albumins übersteigen, d.h. der Marker in einem "Überschuss" bezogen auf die Bindungskapazität des Albumins vorliegen. Die Inkubation der mindestens 2, bevorzugt auch 3, 4, 5, 6, 7 oder 8 Messlösungen führt dazu, dass der Albumin-bindende Marker M an wenigstens einer bestimmten Bindestelle des Albumins binden kann und sich Komplexe aus Marker und Albumin (M:A) bilden. Nach Abtrennen der erzeugten Komplexe (M:A) kann die Menge an ungebundenem Marker M durch Einsatz mindestens eines Teststreifens nachgewiesen werden. Dieser Nachweis muss in mindestens einer der Messlösungen der Test-Probe und der Referenz-Probe erfolgen können. Somit kann nun beispielsweise diejenige Messlösung der mindestens 2, bevorzugt 3, 4, 5, 6, 7 oder 8 Messlösungen der Referenz-Probe bestimmt werden, bei der erstmalig ungebundener Marker M mit mindestens einem Teststreifen detektiert werden kann und diejenige Messlösung der mindestens 2, bevorzugt 3, 4, 5, 6, 7 oder 8 Messlösungen der Test-Probe, bei der letztmalig kein ungebundener Marker mit mindestens einem Teststreifen detektiert werden kann, bestimmt werden. Was genau unter dem Begriff "Teststreifen" zu verstehen ist, wird an anderer Stelle hierin im Detail erläutert. Basierend auf den nachgewiesenen Mengen an ungebundenem Marker M kann nun die relative Bindungskapazität des Albumins, auch relative Bindungsfunktion des Albumins genannt, bestimmt werden, welche Rückschlüsse über den bindungsstellenspezifischen Beladungszustand bzw. die noch vorhandene Bindungskapazität des Albumins an der oder den bestimmten Bindungsstelle(n) erlaubt, was auch an anderer Stelle hierin weiter erläutert wird.

Unter dem Begriff "Bindungskapazität des Albumins" im Sinne der Erfindung versteht man die Fähigkeit des Albumins einen oder mehrere Stoffe, die spezifisch an Albumin binden, aufzunehmen bzw. zu binden. Der Begriff Bindungskapazität umfasst die Aufnahme-Kapazität eines oder mehrerer Stoffe an einer und/oder mehrerer Bindungsstellen. Wenn die Bindungskapazität an einer bestimmten Bindungsstelle, beispielsweise der Bindungsstelle I und/oder II, erreicht ist, kann dies auch als "Sättigung der Bindungsstelle I und/oder II" bezeichnet werden. Wenn beispielsweise ein Stoff wie Diazepam, der an spezifisch an die Bindungsstelle II bindet, die Bindungsstelle II vollständig blockiert, ist die Bindungsstelle II gesättigt und überschüssiges Diazepam, welches noch in der Lösung enthalten ist, kann nicht mehr an Albumin binden. Somit können in besagter Lösung (mit an der Bindungsstelle II gesättigtem Albumin) keine Komplexe aus Albumin und Diazepam mehr gebildet werden und vorhandenes Diazepam verbleibt ungebunden in der Lösung. Die Bindungskapazität des Albumins ist somit als funktionelle Eigenschaft des Albumins anzusehen. Wie bereits oberhalb erwähnt, kann eine große Menge bzw. Konzentration an Albumin beispielsweise eine geringe Bindungsfunktionalität aufweisen ("schlechtes Albumin" für einen Albumin-bindenden Marker), während eine kleine Menge bzw. Konzentration an Albumin eine große Bindungsfunktionalität aufweisen kann ("gutes Albumin" für einen Albumin-bindenden Marker).

Als "Referenz-Bindungskapazität" bezeichnet man eine angenommene oder bekannte Bindungskapazität. Dies kann beispielsweise die Bindungskapazität einer artifiziellen Lösung sein, der Albumin zugesetzt wurde und von der bekannt ist, dass die Bindungsstellen des Albumins nicht belegt sind. Weiterhin kann es sich um eine Probe, bevorzugt eine Serum- oder Plasma-Probe, eines gesunden Probanden handeln, bevorzugt eines Probanden ohne Leberschädigung oder Niereninsuffizienz, von dem bekannt ist, dass keine stärkere Sättigung der Albuminbindungsstellen oder eine Überladung des Albuminmoleküls vorhanden ist. Im Vergleich zu einem kranken Patienten, bevorzugt einem Patienten mit einer Leberschädigung und/oder Niereninsuffizienz ist in der Probe des gesunden Probanden (i.e. in der Referenz-Probe) somit eine bekannte bzw. angenommene höhere Bindungskapazität des Albumins vorhanden als dies bei dem genannten Patienten (i.e. in der Test-Probe) der Fall wäre.

Die "relative Bindungskapazität des Albumins", auch "relative Bindungsfunktion des Albumins" genannt, welche gemäß des erfindungsgemäßen Verfahrens bestimmt wird, erlaubt Aussagen über den bindungsstellenspezifischen Beladungszustand bzw. die noch vorhandene Bindungskapazität des Albumins an der oder den benannten Bindungsstelle(n). Gemäß der Erfindung wird ein Stoff, der an Albumin binden kann, auch als Albumin-Ligand oder Albumin-bindender Marker bezeichnet, wie anderswo hierin beschrieben wird.

Als "Übersteigen der Bindungskapazität" des Albumins bezeichnet man die Tatsache, dass ein Albumin-bindender Marker M nicht mehr an Albumin binden kann und somit die Bindungskapazität des Albumins übersteigt. Wird beispielsweise zu einer Lösung, die Albumin enthält, mehr Marker M zugesetzt als spezifisch an das in der Lösung vorhandene Albumin binden kann, so übersteigt der Marker die Bindungskapazität des Albumins. In der Folge kann in der Lösung (bevorzugt nach Abtrennung von Marker:Albumin Komplexen) ungebundener Marker M detektiert werden, der nicht an Albumin gebunden ist bzw. nicht in Marker:Albumin (M:A) Komplexen vorliegt. Ist beispielsweise die Bindungsstelle II von einem spezifischen Marker wie Diazepam vollständig besetzt, d.h. die Bindungsstelle II ist gesättigt, führt die Zugabe bzw. das Vorhandensein von Diazepam, welches nun nicht mehr an Albumin binden kann (da die Bindungsstelle gesättigt ist) zu einem Übersteigen der Bindungskapazität an der genannten Bindungsstelle II. In einer Lösung mit Albumin, in der die Bindungsstelle II bereits gesättigt ist, können jedoch gegebenenfalls weitere Marker, welche beispielsweise spezifisch an die Bindungsstelle I binden, vom vorhandenen Albumin in der Lösung weiterhin aufgenommen werden, bis die Bindungsstelle I ebenfalls gesättigt ist. Die vermutlich vorhandene Bindungskapazität bzw. ein Übersteigen dieser Bindungskapazität ist somit abhängig vom bereits vorhandenen Beladungszustand des Albuminmoleküls bzw. der Verfügbarkeit von Bindungsstelle(n), an welche der oder die Albumin-bindender(n) Marker binden. Dem Fachmann ist zudem bekannt, dass, auch wenn die Bindungskapazität der einzelnen Bindungsstellen durch allosterische Wechselwirkungen oder strukturelle Veränderungen des Albumin-Moleküls verändert ist, diese Veränderungen der Bindungseigenschaften ebenfalls mit erfasst werden. Wie bereits oberhalb erwähnt, ist anzunehmen, dass die vermutlich vorhandene Bindungskapazität des Albumins in einer Probe eines Patienten mit Leberschädigung und/oder Niereninsuffizienz geringer ist als in einer Probe eines gesunden Probanden, da eine Leberschädigung und/oder Niereninsuffizienz bekanntermaßen zu einer stärkeren Sättigung bzw. Überladung des Albumins führen kann.

Darüber hinaus soll das Übersteigen der Bindungskapazität des Albumins eines Albumin-bindenden Markers M gemäß der Erfindung dazu führen, dass das Vorhandensein von "überschüssigem" bzw. ungebundenem Marker M in der Probe, d.h. ungebundener Marker M in mindestens einer Messlösung der Test-Probe und der Referenz-Probe nach Abtrennen der Marker:Albumin Komplexe (wie anderswo hierin im Detail beschrieben), nachweisbar ist. Bevorzugt weist der überschüssige bzw. ungebundene Marker M, besonders bevorzugt der ungebundene Marker Diazepam, eine Konzentration von mindestens 100ng/ml, mindestens 200ng/ml, mindestens 300ng/ml, mindestens 400ng/ml oder mindestens 500ng/ml auf.

Unter "Albumin-bindender Marker M" gemäß der Erfindung versteht man einen Stoff, der spezifisch an Albumin binden kann. Der Albumin-bindender Marker M kann hierbei an einer oder mehrerer Bindungsstellen des Albumins binden. Gemäß der Erfindung bindet der Albumin-bindende Marker M bevorzugt an die Bindungsstelle I und/oder II des Albumins. Dies umfasst beispielsweise dem Fachmann bekannte Stoffe, von denen gezeigt wurde, dass sie an die jeweiligen Bindungsstellen des Albumins binden können wie beispielsweise Diazepam, andere Benzodiazepine, Tryptophan, Gallensäuren, Dansylsarcosin, mittelkettigen Fettsäuren, Warfarin, Furosemid, Sulfonylharnstoffe und Dansylamid oder auch Opioide wie Fentanyl, synthetische Drogen wie Kokain oder Cannabinnoide.

Im Sinne der Erfindung ist der Albumin-bindende Marker M ein Benzodiazepin, Tryptophan, eine Gallensäure, Dansylsarcosin, eine mittelkettige Fettsäure, Warfarin, Furosemid, ein Sulfonylharnstoff, Dansylamid, ein Opioid, Kokain oder ein Cannabinoid; besonders bevorzugt Diazepam. Das erfindungsgemäße Verfahren soll mindestens einen Albumin-bindenden Marker M umfassen. Der Einsatz mehrerer Marker ist jedoch ebenfalls bevorzugt, beispielsweise kann eine Kombination von Markern, die an verschiedenen Bindungsstellen des Albuminmoleküls binden oder eine Kombination von Markern, die an der gleichen Bindungsstelle, beispielsweise an der Bindungsstelle I des Albumins binden, eingesetzt werden. Marker, die an der gleichen Bindungsstelle binden, können wiederum Stoffe sein, die mit gleicher, ähnlicher oder unterschiedlicher Bindungsstärke an bestimmte Bindungsstelle(n), beispielsweise die Bindungsstelle I des Albumins, binden.

Der Begriff "Bindungsstelle I" bzw. "Bindungsstelle II" des Albumins bezeichnet zwei Bindungsstellen des Albumins, an die Substanzen spezifisch binden können. Es ist dem Fachmann bekannt, dass neben 7 Bindungsstellen für langkettigen Fettsäuren und einer freien SH-Gruppe am Cystein 34, beispielsweise für Stickstoffmonoxid, zwei gruppenspezifische Bindungsstellen des Albumins für die Vielzahl von körpereigenen (endogenen) oder applizierten (exogenen) Substanzen zur Verfügung stehen. Bezug nehmend auf die Arbeiten zur Charakterisierung der Albuminbindungsstellen von Sudlow und Kollegen werden diese als Bindungsstellen I und II bezeichnet (Sudlow G, et.al., The characterization oftwo specific drug binding sites on human serum albumin. Mol Pharmacol 1975; 11(6): 824-832). Es ist dem Fachmann zudem bekannt, dass die Bindungsstelle I des Albumins oftmals auch als Warfarin/Bilirubin-Bindungsstelle bezeichnet wird und hauptsächlich heterozyklische Substanzen oder Dicarboxyl-Säuren bindet, während die Bindungsstelle II oftmals auch als Diazepam/Indol-Bindungsstelle bezeichnet wird und hauptsächlich Liganden mit einer aromatischen Grundstruktur bindet. Beispielsweise binden an die Bindungsstelle I bevorzugt Warfarin, Furosemid oder Dansylamid, während an die Bindungsstelle II bevorzugt Indole, wie Tryptophan oder auch Diazepam, Gallensäuren, Dansylsarcosin und mittelkettige Fettsäuren binden (Peters T. All about albumin : biochemistry, genetics, and medical applications. Academic Press, 1996; Ghuman J et al, Structural basis of the drug-binding specificity of human serum albumin. J Mol Biol (2005); 353: 38-52).

Gemäß der Erfindung soll das Inkubieren der Messlösungen unter Bedingungen stattfinden, die es erlauben, dass der mindestens eine Albumin-bindende Marker M an Albumin binden kann, sodass Komplexe aus diesem Marker M und Albumin (M:A) entstehen. Bevorzugt handelt es sich hierbei um Komplexe aus einem Benzodiazepin, bevorzugt Diazepam, und Albumin. Bevorzugt soll das Inkubieren der Messlösungen bei Raumtemperatur für bis zu 30 Minuten, besonders bevorzugt für mindestens 10 Sekunden, 20 Sekunden, 45 Sekunden, 1 Minute, 2 Minuten, 5 Minuten, 10 Minuten, 15 Minuten, 20 Minuten oder 30 Minuten stattfinden.

Unter "Abtrennen der Komplexe" versteht man die Separation der Komplexe aus mindestens einem Albumin-bindende Marker M und Albumin (M:A Komplexe) von der Messlösung. Methoden und Mittel um M:A Komplexe, bevorzugt Diazepam-Albumin Komplexe, abzutrennen sind dem Fachmann bekannt und umfassen beispielsweise Zentrifugation, Filtration oder spezifische Adsorptionsmethoden wie Immunadsorption. Nach dem Abtrennen der M:A Komplexe verbleibt eine Lösung, beispielsweise ein albuminfreies Filtrat, in dem keine M:A Komplexe mehr vorhanden sind. In dieser Lösung kann nun das Vorhandensein bzw. die Menge an ungebundenen Marker M ermittelt werden.

Der Begriff "Nachweis" wie hier verwendet umfasst die qualitative, semi-quantitative und/oder quantitative Bestimmung der Anwesenheit von ungebundenen Marker M in einer Lösung. Unter dem Begriff "Nachweisen des Vorhandensein oder der Menge an ungebundenem Marker M" versteht man das Nachweisen an ungebundenem Marker M in mindestens einer Messlösung der Test-Probe bzw. der Referenzprobe nach Abtrennen der M:A Komplexe (wie oberhalb erläutert). Der Begriff Menge ist hierbei nicht im Sinne einer absoluten Menge zu verstehen, sondern bevorzugt als semi-quantitative Aussage, beispielsweise im Sinne einer Mindestmenge ab der das Vorhandensein des ungebundenen Markers anhand der Detektionsgrenze des eingesetzten Teststreifens möglich ist. Das Nachweisen des Vorhandenseins oder der Menge an ungebundenem Marker M umfasst bevorzugt die Bestimmung des qualitativen Vorhandenseins an ungebundenem Marker, d.h. eine Ja oder Nein Aussage, ob ungebundener Marker in der Lösung, in die der Teststreifen getaucht wird, vorhanden ist.

Unter dem Begriff "Detektionsgrenze", auch als Nachweisgrenze bezeichnet, versteht man den extremen Wert eines Messverfahrens, bis zu dem die Messgröße gerade noch zuverlässig nachgewiesen werden kann. Wird beispielsweise ein Teststreifen für Diazepam mit einer vorbestimmten Detektionsgrenze von 100ng/ml eingesetzt, so ist es bei einem entsprechendem "positiven" Signal des Teststreifens möglich, die Aussage zu treffen, dass in der besagten Lösung ungebundener Marker in einer Mindestmenge von 100ng/ml vorhanden ist bzw. bei einem entsprechenden "negativen" Signal des Teststreifens die Aussage zu treffen, dass kein ungebundener Marker vorhanden ist oder die Menge an ungebundenem Marker zu gering ist, um detektiert zu werden. Es ist dem Fachmann hierbei bekannt, dass die entsprechenden Signale, die jeweils als positiv oder negativ anzusehen sind, abhängig von der zu detektierenden Substanz und/oder dem eingesetzten Teststreifen sind und/oder auf dem Nachweisverfahren des Teststreifens beruhen. Gemäß der Erfindung liegt die vorbestimmte Detektionsgrenze bevorzugt bei mindestens 1 ng/ml, 10ng/ml, 50ng/ml, 100ng/ml, 200ng/ml, 300ng/ml, 400ng/ml oder 500ng/ml.

Unter dem Begriff "Toleranzgrenze", auch als "cut-off" bezeichnet, versteht man den Wert oder Grenzbereich, ab wann ab wann ein Testergebnis als positiv bzw. negativ zu bewerten ist. Um die Nachweissicherheit eines Tests bzw. eines Teststreifens zu gewährleisten und somit "falsch positive" Resultate zu vermeiden, liegt die Toleranzgrenze (cut-off) üblicherweise um ein Mehrfaches oberhalb der Detektionsgrenze. Die Toleranzgrenze kann allerdings auch mit der Detektionsgrenze übereinstimmen. Üblicherweise erfolgt bei einem Streifentest ein sogenanntes "positives Signal", wenn die Marker-Konzentration, beispielsweise die Menge an ungebundenem Diazepam in der Lösung nach Abtrennen der M:A Komplexe, unterhalb der angegebenen Toleranzgrenze (beispielsweise einem cut-off Wert von 200ng/ml) liegt. Bevorzugte Toleranzgrenzen für Teststreifen gemäß der Erfindung, bevorzugt für ein Benzodiazepin, besonders bevorzugt für Diazepam, sind etwa 100ng/ml, 200ng/ml, 300ng/ml, 400ng/ml, 500ng/ml und 600ng/ml.

Es ist dem Fachmann zudem bekannt, dass es für einzelne Substanzen bzw. Teststreifen verschiedene bevorzugte Toleranzgrenzen gibt. Beispielsweise können die Toleranzgrenzen von der zu detektierenden Substanz und/oder dem eingesetzten Teststreifen und/oder dem Nachweisverfahren des Teststreifens abhängig sein. Zudem können auch regulatorische Aspekte eine Rolle spiele, beispielsweise Richtlinien und Vorgaben der US-amerikanischen Drogenbehörde (NIDA) für die Zulassung von Teststreifen für den Nachweis von Drogen wie Kokain oder Amphetamin. Hierbei soll sichergestellt werden, dass die Entscheidung, i.e. ab wann ein Testergebnis als positiv bzw. negativ zu bewerten ist, speziellen Anforderungen genügt. Beispielsweise soll ein Teststreifen für Opiate nicht so eingestellt sein, dass bereits der Verzehr eines Mohnbrötchens ein positives Signal ergibt, sondern das Vorhandensein von Opiaten erst ab einer gewissen Menge als positiv zu werten ist. Für übliche Drogentests bzw. entsprechende Teststreifen, die von der NIDA zugelassen sind, liegt die Toleranzgrenze oftmals ein Vielfaches, e.g. hundert bis tausendfach, über der Detektionsgrenze der nachzuweisenden Substanz. Teststreifen mir unterschiedlichen Toleranzgrenzen (cut-offs) für verschiedene Substanzen sind dem Fachmann bekannt. Toleranzgrenzen zum Nachweis von Amphetamin liegen beispielsweise bevorzugt bei etwa 300ng/ml, 500ng/ml, 1000ng/ml oder 3000ng/ml, für Kokain bei etwa 100ng/ml, 150ng/ml, 300ng/ml oder 400ng/ml, für Fentanyl bei etwa 20ng/ml oder 100ng/ml, für Opiate wie Morphin bei etwa 300ng/ml, 1000ng/ml oder 2000ng/ml, für Marihuana (THC) bei etwa 20ng/ml, 50ng/ml, 150ng/ml oder 500ng/ml, für Oxycodon bei etwa 100ng/ml oder 200ng/ml, für Phencylidin bei etwa 25ng/ml oder 50ng/ml und für Metamphetamin bei etwa 300ng/ml, 500ng/ml oder 1000 ng/ml.

Der Begriff "Teststreifen" umfasst jegliche Teststreifen oder Streifentests, mit der Nachweis eines spezifischen Markers, bevorzugt eines Albumin-bindender Marker M, erfolgen kann. Bevorzugt handelt es sich um Teststreifen für den Einsatz in wässrigen Lösungen, bevorzugt mit einem pH-Wert zwischen 5 und 8, besonders bevorzugt mit einem pH-Wert zwischen 7 und 8. Teststreifen zum Nachweis für Benzodiazepine sind dem Fachmann bekannt. Darüber hinaus ist dem Fachmann bekannt, dass - je nach eingesetztem Teststreifen - die Anwesenheit oder die Abwesenheit eines Signals ein positives Ergebnis bedeuten kann. Beispielsweise kommt bei kommerziell erhältlichen Teststreifen für Benzodiazepine häufig ein kompetitiver Immunassay zum Einsatz. Hierbei wird zur Detektion kein zweiter, markierter Antikörper verwendet, sondern ein markiertes Kompetitor-Antigen (eine synthetische Verbindung, die dem Analyten, beispielsweise Diazepam, strukturell ähnlich ist und auch am Antikörper bindet) eingesetzt. So kommt es zur Kompetition (Konkurrenz) zwischen Analyt (Diazepam) und Kompetitor um einen Bindungsplatz am Antikörper. Das Signal verhält sich hier umgekehrt zur Analyt-Konzentration, d.h. wenig Analyt = fast alle Antikörperbindestellen werden von markiertem Kompetitor besetzt => starke Farbreaktion; viel Analyt => schwache Farbreaktion. Im Sinne der Erfindung handelt es sich bevorzugt um einen Teststreifen, der auf einem immunchemischen Test zum schnellen Nachweis eines Albumin-bindenden Markers mit visuell ablesbarem Ergebnis, beruht. Beispielsweise ein Teststreifen, der auf einem kompetitiven Sandwich-ELISA beruht und den Nachweis von Benzodiazepinen mit einem cut-off von 300ng/ml mittels visuell ablesbarer Kontroll- und Testlinie erlaubt. Ist hierbei ein Benzodiazepin in der Lösung vorhanden, erscheint eine farbige Linie (Kontrolllinie). Ist kein Benzodiazepin in der Lösung vorhanden, so erscheinen zwei farbige Linien (Test und Kontrolllinie).

Gemäß der Erfindung soll der mindestens eine Teststreifen die Bestimmung von ungebundenem Marker M in den Lösungen nach Abtrennen der Komplexe aus Marker und Albumin (M:A), wie anderswo hierin beschrieben, erlauben. Bevorzugt ist weiterhin der Einsatz von mehr als einem Teststreifen, bevorzugt 3, 4, 5 oder 6 Teststeifen, welche unterschiedliche vorbestimmte Detektionsgrenzen und/oder unterschiedliche vorbestimmte Toleranzgrenzen für einen oder mehrere Albumin-bindende(n) Marker M aufweisen.

Wie bereits oberhalb erwähnt, erfolgt bei den meisten Teststreifen ein sogenanntes "positives Signal", wenn die Markerkonzentration, beispielsweise die Menge an ungebundenem Diazepam in der Lösung nach Abtrennen der M:A Komplexe, unterhalb der angegebenen Toleranzgrenze, beispielsweise einem cut-off Wert von 200ng/ml, liegt. Werden nun beispielsweise zwei Teststreifen in der besagten Lösung eingesetzt, wobei der erste Teststreifen einen cut-off von 200ng/ml und der zweite einen cut-off von 300ng/ml aufweist, so ist die direkte, semi-quantitive Abschätzung der freien Diazepam-Konzentration in der Lösung möglich. In diesem Beispiel beträgt die freie Diazepam-Konzentration demnach weniger als 200 ng/ml, wenn in beiden eingesetzten Teststreifen ein Signal generiert wird; ist bei beiden Teststreifen kein Testsignal zu detektieren, liegt die Diazepam-Konzentration in der Lösung oberhalb von 300 ng/ml. Ist jedoch auf dem Testreifen mit 300 ng/ml cut-off ein Signal vorhanden, auf dem Teststreifen mit dem 200 ng/ml cut-off jedoch nicht, kann die Diazepamkonzentration in der Lösung im Bereich zwischen 200 und 300 ng/ml angegeben werden. Durch geeignete Auswahl und Kombinationen der Teststreifen ist somit eine semiquantitative Abschätzung der Diazepam-Konzentration in der genannten Lösung möglich und eine Bestimmung der relativen Albumin-Bindungs-Funktion kann entsprechend erfolgen. Gemäß der Erfindung kommen bevorzugt Teststreifen zum Einsatz, die den Nachweis von Benzodiazepinen, besonders bevorzugt Diazepam, oder von synthetischen Drogen wie Amphetamine, Cannabis. Methadon oder Opiaten erlauben. Bevorzugt sind weiterhin Teststreifen mit unterschiedlichen vorbestimmte Detektionsgrenzen und/oder unterschiedliche vorbestimmten Toleranzgrenzen für einen oder mehrere Albumin-bindende(n) Marker M, die auf einem gemeinsamen Träger zusammengefasst sind.

Die "relative Bindungskapazität des Albumins" kann auch als relative Albumin-Bindungsfunktion (rABFx) oder "ABiC" gemäß der englischen Übersetzung "Albumin Binding Capacity" bezeichnet werden. Das Bestimmen der relativen Bindungskapazität des Albumins basiert erfindungsgemäß auf den mittels Teststreifen nachgewiesenen Mengen an ungebundenem Marker in den Lösungen nach Abtrennen der Marker-Albumin (M:A) Komplexe.

Bevorzugt wird die relative Bindungskapazität des Albumins (rABFx), entsprechend der folgenden Formel bestimmt:

$$ \mathrm{rABF}\ x = \frac{mV_P}{mV_R} $$

wobei x als Kennzeichnung der spezifischen Bindungsstelle (beispielsweise Bindungsstelle I oder II) dient und mVR bzw. mVP diejenige Messlösung bzw. molaren Verdünnungsstufe oder Verhältnisstufe von Marker zu Albumin (mV) darstellt, bei der in der Referenz (mVR) erstmalig ungebundener Marker bzw. in der Probe (mVP) letztmalig kein ungebundener Marker gemäß der Erfindung mittels Teststreifen detektiert werden kann. Wenn die molare Verdünnungsstufe bzw. Messlösung, bei der erstmalig ungebundener Marker im Filtrat detektiert werden kann, in der Probe kleiner als die der Referenz ist, liegt der Wert der relativen Bindungskapazität des Albumins unter 1. Sind beide molaren Verdünnungsstufen gleich, beträgt die relative Bindungskapazität des Albumins 1.

Gegenüber den im Stand der Technik bekannten Verfahren ist das erfindungsgemäße Verfahren schnell, effizient, kostengünstig und kann ohne besondere infrastrukturelle Voraussetzungen eingesetzt werden. Durch das erfindungsgemäße Verfahren ist eine Bestimmung der Bestimmung der relativen Bindungskapazität des Albumins in der Nähe des Patienten, also eine Point-of-care Diagnostik, möglich. Dadurch kann der gegenwärtige Zustand des Patienten bei der Therapie-Planung berücksichtigt werden, beispielsweise kann die Gabe von Infusionen, Dosierung von Medikamenten, Beginn, Dauer und Intensität extrakorporaler Verfahren entsprechend angepasst werden, sodass Nebenwirkungen einer Übertherapie oder die Gefahr einer Unterversorgung des Patienten reduziert werden.

Die zuvor getroffenen Definitionen und gemachten Erklärungen der Begriffe gelten für die nachfolgend beschriebenen Ausführungsformen entsprechend.

In einer bevorzugten Ausführungsform des erfindungsgemäßen Verfahrens bindet der mindestens eine Albumin-bindende Marker M an die Bindungsstelle I und/oder II des Albumins.

In einer anderen bevorzugten Ausführungsform des erfindungsgemäßen Verfahrens ist der mindestens eine Albumin-bindende Marker M ein Benzodiazepin. Besonders bevorzugt ist der mindestens eine Albumin-bindende Marker M Diazepam.

In einer weiteren bevorzugten Ausführungsform des erfindungsgemäßen Verfahrens weist der der mindesten eine Teststreifen eine vorbestimmte Detektionsgrenze und/oder eine vorbestimmte Toleranzgrenze für den mindestens einen Albumin-bindende Marker M auf.

In einer weiteren bevorzugten Ausführungsform des erfindungsgemäßen Verfahrens werden mehrere Teststreifen für unterschiedliche Albumin-bindende Marker M eingesetzt.

In einer weiteren bevorzugten Ausführungsform des erfindungsgemäßen Verfahrens liegt die definierte Detektionsgrenze für den Albumin-bindenden Marker M bei mindestens 100ng/ml liegt und/oder eine vorbestimmte Toleranzgrenze bei etwa 200ng/ml.

In einer weiteren bevorzugten Ausführungsform des erfindungsgemäßen Verfahrens ist die Test-Probe eine Probe aus einem Patienten mit einer Leberschädigung und/oder einer Niereninsuffizienz und/oder einer Sepsis oder eine Albumin-haltige Lösung.

In einer weiteren bevorzugten Ausführungsform des erfindungsgemäßen Verfahrens ist die Referenz-Probe eine Probe eines gesunden Probanden oder eine artifiziell hergestellte Albumin-Lösung.

Die Erfindung betrifft weiterhin ein Verfahren zur Bestimmung der Menge an funktionellem Albumin, umfassend:

a) Bereitstellen einer Test-Probe mit einer bestimmten Menge an Albumin mit unbekannter Bindungskapazität und einer Referenz-Probe mit der gleichen bestimmten Menge an Albumin mit einer Referenz-Bindungskapazität;

b) Inkubieren der Test-Probe und der Referenz Probe mit einer definierten Menge mindestens eines Albumin-bindenden Markers M zur Test-Probe und zur Referenz-Probe unter Bedingungen, die es erlauben, dass der mindestens eine Albumin-bindende Marker M an Albumin binden kann, sodass Komplexe aus diesem Marker M und Albumin (M:A) entstehen;

c) Abtrennen der in Schritt c) erzeugten Komplexe (M:A);

d) Nachweisen der Menge an ungebundenem Marker M in den Proben nach Abtrennen des Komplexes (M:A) durch einen ersten und einen zweiten Teststreifen, die die Bestimmung der Menge des ungebundenen Markers erlauben, wobei die Teststreifen unterschiedliche vorbestimmte Toleranzgrenzen aufweisen; und

e) Bestimmen der Menge an funktionellem Albumin basierend auf den nachgewiesenen Mengen des Markers M in Schritt d) wobei der mindestens eine Albumin-bindende Marker M ein Benzodiazepin, Tryptophan, eine Gallensäure, Dansylsarcosin, eine mittelkettige Fettsäure, Warfarin, Furosemid, ein Sulfonylharnstoff, Dansylamid, ein Opioid, Kokain oder ein Cannabinoid ist.

Bei der Test-Probe und der Referenz-Probe im Sinne der Erfindung handelt es sich um eine Lösung, die Albumin enthält, wie anderswo hierin im Detail erläutert. Es handelt sich zudem um ein *in vitro* Verfahren. Das Bereitstellen einer Probe umfasst erfindungsgemäß kein Verfahren, das am menschlichen Körper vorgenommen wird.

In einer bevorzugten Ausführungsform des erfindungsgemäßen Verfahrens weist der erste Teststreifen eine vorbestimmte Toleranzgrenze, besonders bevorzugt für Diazepam, von etwa 200ng/ml auf und/oder der zweite Teststreifen eine vorbestimmte Toleranzgrenze von etwa 300ng/ml auf.

Weiterhin bevorzugt ist eine Toleranzgrenze für den ersten Teststreifen von etwa 300ng/ml und eines zweiten Teststreifens von etwa 500ng/ml für den Nachweis eines Amphetamins, eine Toleranzgrenze für den ersten Teststreifen von etwa 100ng/ml und eines zweiten Teststreifens von etwa 300ng/ml für den Nachweis von Kokain, eine Toleranzgrenze für den ersten Teststreifen von etwa 20ng/ml und eines zweiten Teststreifens von etwa 100ng/ml für den Nachweis von Fentanyl, eine Toleranzgrenze für den ersten Teststreifen von etwa 300ng/ml und eines zweiten Teststreifens von etwa 1000ng/ml für den Nachweis eines Opiates wie Morphin, eine Toleranzgrenze für den ersten Teststreifen von etwa 20ng/ml und eines zweiten Teststreifens von etwa 150ng/ml für den Nachweis von Marihuana (THC), eine Toleranzgrenze für den ersten Teststreifen von etwa 100ng/ml und eines zweiten Teststreifens von etwa 200ng/ml für den Nachweis von Oxycodon, eine Toleranzgrenze für den ersten Teststreifen von etwa 25ng/ml und eines zweiten Teststreifens von etwa 50ng/ml für den Nachweis von Phencylidin, eine Toleranzgrenze für den ersten Teststreifen von etwa 300ng/ml und eines zweiten Teststreifens von etwa 500ng/ml für den Nachweis eines Metamphetamins.

Bevorzugt ist weiterhin der Einsatz von mehr als einem Teststreifen, bevorzugt 3, 4, 5 oder 6 Teststeifen, welche unterschiedliche vorbestimmte Detektionsgrenzen und/oder unterschiedliche vorbestimmte Toleranzgrenzen für einen oder mehrere Albumin-bindende(n) Marker M aufweisen. Gemäß der Erfindung kommen bevorzugt Teststreifen zum Einsatz, die den Nachweis von Benzodiazepinen, besonders bevorzugt Diazepam, oder von synthetischen Drogen wie Amphetamine, Cannabis. Methadon oder Opiaten erlauben. Bevorzugt sind weiterhin Teststreifen mit unterschiedlichen vorbestimmte Detektionsgrenzen und/oder unterschiedliche vorbestimmten Toleranzgrenzen für einen oder mehrere Albumin-bindende(n) Marker M, die auf einem gemeinsamen Träger zusammengefasst sind.

Erfindungsgemäß wird die Menge an ungebundenem Marker M in den Proben nach Abtrennen des Komplexes (M:A) mittels mindestens zwei Teststreifen ermittelt, die unterschiedliche Toleranzgrenzen aufweisen. Bevorzugt ist weiterhin der Einsatz von mehr als zwei Teststreifen, bevorzugt 3, 4, 5, 6, 7 oder 8 Teststreifen mit unterschiedlichen Toleranzgrenzen bzw. die Kombinationen mehrerer Streifentests mit unterschiedlichen Toleranzgrenzen (cut-off Werten).

Beispielsweise kann durch den Einsatz mehrerer für den Marker M (bevorzugt Diazepam) spezifischen Teststreifen mit unterschiedlichen Cut-off-Werten wie 100ng/ml, 200ng/ml, 300ng/ml, 400ng/ml und 500 ng/ml, welche auf einem Träger zusammengefasst sind, in einer Probe als auch zu einer Referenz-Lösung (die beide die ungefähr gleiche Albuminkonzentration und die ungefähr gleiche Menge an Albumin-bindendem Marker M aufweisen), die Menge an ungebundenem Marker M bestimmt werden und somit auf den Anteil an funktionellem Albumin bzw. auf die Bindungsfunktion des Albumins geschlossen werden.

Die Bestimmung der relativen Bindungskapazität bzw. die Berechnung der relativen Albumin-Bindungsfunktion (rABFx) kann bevorzugt entsprechend der folgenden Formel bestimmt werden:

$$\mathrm{rABF}\,\mathrm{x} = \frac{C_R}{C_P}$$

mit x als Kennzeichnung der spezifischen Bindungsstelle (z.B. I oder II) und CP bzw. CR als Menge bzw. Konzentration des ungebundenen Markers nach Schritt d) des erfindungsmäßen Verfahrens in der Probe (CP) und der Referenz (CR), beispielsweise der Diazepamkonzentration im Filtrat nach Abtrennen der Marker Albumin:Marker (M:A) Komplexe der Test-Probe und der Referenz-Probe gemäß der Erfindung.

In einer weiteren bevorzugten Ausführungsform des erfindungsgemäßen Verfahrens bindet der mindestens eine Albumin-bindende Marker M an die Bindungsstelle I und/oder II des Albumins.

In einer anderen bevorzugten Ausführungsform des erfindungsgemäßen Verfahrens ist der mindestens eine Albumin-bindende Marker M ein Benzodiazepin. Besonders bevorzugt ist der mindestens eine Albumin-bindende Marker M Diazepam.

In einer weiteren bevorzugten Ausführungsform des erfindungsgemäßen Verfahrens ist die Test-Probe eine Probe aus einem Patienten mit einer Leberschädigung und/oder einer Niereninsuffizienz und/oder einer Sepsis oder eine Albumin-haltige Lösung.

In einer weiteren bevorzugten Ausführungsform des erfindungsgemäßen Verfahrens ist die Referenz-Probe eine Probe eines gesunden Probanden oder eine artifiziell hergestellte Albumin-Lösung.

Weiterhin umfasst die Erfindung ein Verfahren, bei welchem der Nachweis des Vorhandenseins bzw. der Menge an ungebundenem Marker M direkt in der Test-Probe und der Referenz-Probe erfolgen kann, ohne dass ein Separationsschritt bzw. ein Abtrennens von Albumin:Marker (M:A) Komplexen erfolgen muss. Dies wird durch den Einsatz mindestens eines Teststreifens, der spezifisch mit dem ungebundenen Marker M reagiert, erreicht. Beispielsweise kann eine Ummantelung des oder der Teststreifen(s) mit einer künstlichen oder biologischen Membran mit einer definierten Porengröße erfolgen, sodass nur die ungebundenen Markermoleküle in direkten Kontakt mit dem Teststreifen treten können, während die an Albumin gebundenen Markermoleküle aufgrund der Größe des Albuminmoleküls zurückgehalten und somit nicht detektiert werden können.

ABBILDUNGEN

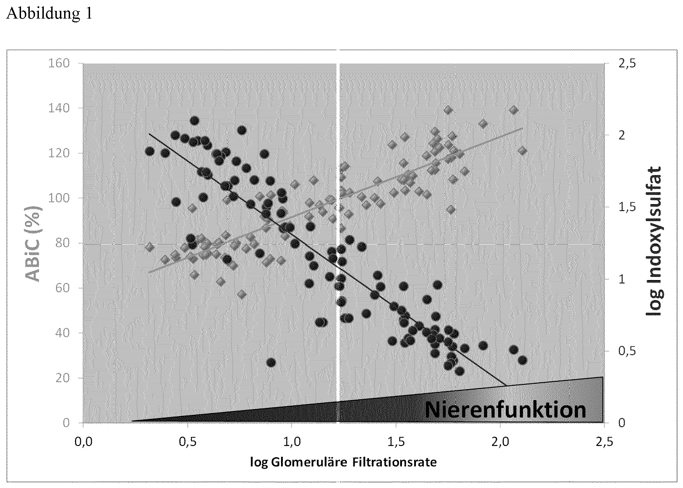

**Abbildung 1:** Abnehmende Albuminfunktion und zunehmende Urämietoxinbeladung mit zunehmender Schwere des Nierenversagens

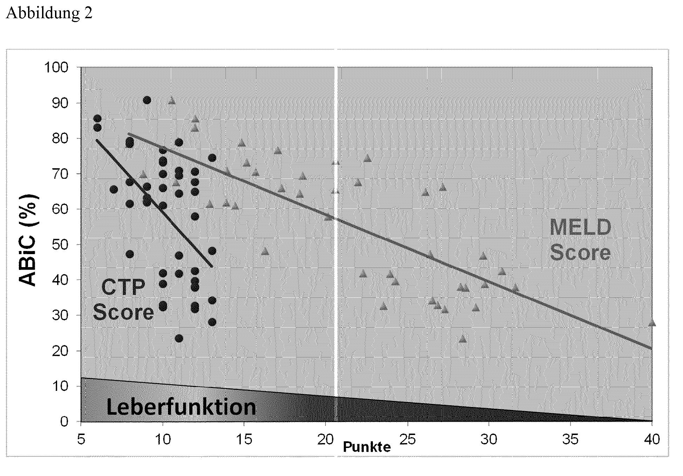

**Abbildung 2:** Abnehmende Albuminfunktion mit zunehmender Schwere des Leberversagens, dargestellt anhand der klinischen Einteilungssysteme CHILD-Klassifikation und MELD

**Abbildung 3:** Bestimmung der relativen Bindungskapazität des Albumins in Plasmaproben von Patienten mit einer chronischen Leberschädigung (Patient 4,5,6) und Patienten mit einer terminalen Niereninsuffizienz (Patient 1,2,3), einer stabilisatorenhaltigen pharmazeutischen Albuminpräparation, sowie Plasma eines gesunden Freiwilligen. A) Diazepamkonzentration in der Probe ($\mu$mol/l), bei der letztmalig noch kein freies Diazepam im Filtrat nachweisbar war (cut-off 200ng/ml = 0,7 $\mu$mol/l). B) Releative Albumin-Bindungsfunktion der Bindungsstelle II (rABFII).

BEISPIELE

Die nachfolgenden Beispiele dienen der Illustration der Erfindung. Sie dürfen im Hinblick auf den Schutzumfang nicht in einschränkender Weise ausgelegt werden.

**Beispiel 1:** Prinzip der Bestimmung der relativen Albumin-Funktion (rABF) bzw. der relativen Bindungskapazität des Albumins

Zu einer albuminhaltigen Probe P wird ein spezifischer Marker M, der an das Albuminmolekül bindet, hinzugegeben und die ungebundene Markermenge MP mittels eines Streifentestes quantifiziert. Parallel wird zu einer albuminhaltigen Referenzlösung R mit der gleichen Albuminkonzentration wie die Probe P die gleiche Menge des spezifischen Markers M hinzugegeben und auch in der Referenzlösung die ungebundene Markermenge MR detektiert.

**Beispiel 2:** Detektion des Vorhandenseins von ungebundenen Marker mittels eines Markerspezifischen Streifentests

Sowohl zu mehreren Aliquots einer Probe P als auch einer Referenz R, die alle die gleiche Albuminkonzentration aufweisen, werden unterschiedliche Markermengen gegeben, so dass absteigende molare Verhältnisstufen Marker/Albumin in der Probe und der Referenz entstehen (z.B. 0,3; 0,25; 0,2; 0,15, 0,1 und 0,05). Nach einem Separationsschritt wird das Vorhandensein des ungebundenen Markers in den albuminfreien Filtraten der einzelnen Verhältnisstufen der Probe als auch der Referenz mittels eines markerspezifischen Teststreifens mit definierten cut-off (z.B. 200 ng/ml für Diazepam) analysiert. Die relative Albumin-Bindungsfunktion (rABF) wird entsprechend der folgenden Formel bestimmt:

$$\text{rABF x} = \frac{mV_P}{mV_R}$$

Mit: x als Kennzeichnung der spezifischen Bindungsstelle (z.B. I oder II) und m VR bzw. m VP als derjenigen molaren Verdünnungsstufe, bei der in der Referenz bzw. in der Probe erstmalig ungebundener Marker bzw. letztmalig kein ungebundener Marker im Filtrat mittels Streifentest detektiert werden kann.

Wenn die molare Konzentrationsstufe, bei der erstmalig ungebundener Marker im Filtrat detektiert werden kann, in der Probe kleiner als die der Referenz ist, liegt der Wert der relativen Albumin-Bindungsfunktion unter 1. Sind beide molaren Verdünnungsstufen gleich, beträgt die relative Albumin-Bindungsfunktion 1.

**Beispiel 3:** Verfahren zur Bestimmung der Menge an funktionellem Albumin bzw. Detektion der ungebundenen Markermenge mittels Kombinationen von Streifentesten mit unterschiedlichen cut-off Werten

Sowohl zu einer Probe P als auch zu einer Referenz R, die beide die gleiche Albuminkonzentration aufweisen, wird die Markermenge M gegeben und nach einem Separationsschritt die Konzentration des ungebundenen Markers durch den Einsatz von markerspezifischen Teststreifen mit unterschiedlichen Cut-off-Werten ( z.B. 100, 200, 300, 400, 500 ng/ml für Diazepam) im albuminfreien Filtrat sowohl der Referenz als auch der Probe bestimmt und die relative Albumin-Bindungsfunktion (rABF) entsprechend der folgenden Formel bestimmt:

$$\mathrm{rABF\ x} = \frac{C_R}{C_P}$$

Mit: x als Kennzeichnung der spezifischen Bindungsstelle (z.B. I oder II) und $C_R$ bzw. $C_P$ als Diazepamkonzentration im Filtrat der Probe bzw. der Referenz.
Die Detektion der ungebundenen Markermenge mittels markerspezifischen Streifentest könnte auch in der Probe P und der Referenz erfolgen (ohne Separationsschritt), wenn sichergestellt werden kann, dass nur die ungebundene Markermenge durch den Streifentest detektiert werden kann. Dieses könnte z.B. durch Ummantelung des oder der Teststreifen(s) mit einer Membran (künstlich oder biologisch) mit einer definierten Porengröße erfolgen, die nur die ungebundenen Markermoleküle in direkten Kontakt mit dem Teststreifen treten läßt, während die an Albumin gebundenen Markermoleküle aufgrund der Größe des Albuminmoleküls zurückgehalten und nicht detektiert werden können.

**Beispiel 4:** Bestimmung der relativen Bindungskapazität des Albumins in Plasmaproben von Patienten mit einer chronischen Leberschädigung und Patienten mit einer terminalen Niereninsuffizienz

Eine Plasmaprobe wird derart aliquotiert und entsprechend PBS hinzugegeben, dass im Ergebnis 8 Aliquots mit einem Volumen von 0,9 ml und einer Albuminkonzentration von 83,3 μmol/l vorliegen. Zu diese Aliquots werden jeweils 0,1 ml von Diazepam-haltigen Lösungen unterschiedlicher Diazepamkonzentration gegeben, dass im Ergebnis 6 Aliquots mit einer Albuminkonzentration von 75 μmol/l und Diazepamkonzentrationen von 18; 15; 12; 9; 6 und 3 μmol/l (entsprechend einem molaren Verhältnis von Diazepam zu Albumin von 0,24; 0,2; 0,16; 0,12; 0,08 und 0,04) vorliegen.
Nach einer Inkubationszeit werden durch Zentrifugation (Centrisart Sartorius, cut off 20 000 Dalton) die ungebundenen Markermengen separiert. Nachfolgend wird die Menge Diazepam im Filtrat mittels Streifentest bestimmt (Teststreifen cut off 200 ng/ml). Bei dem eingesetzten Streifentest erfolgt ein Signal, wenn die Diazepamkonzentration in der Flüssigkeit (Filtrat) unterhalb des angegebene cut off Wertes (200 ng/ml) liegt. Es wird die Probe (bzw. diejenige Diazepam-Konzentrationsstufe) ermittelt, bei der letzmalig das Signal noch festzustellen ist, d.h. die Konzentration im Filtrat letzmalig unterhalb des cut off Wertes liegt.
Das gleiche wird mit einer Referenzprobe (z.B. gesunde Kontrolle) durchgeführt und auch hier diejenige Diazepamkonzentrationsstufe ermittelt, bei der das Signal des Teststreifens im Ultrafiltrat letzmalig vorhanden ist. Diese beiden Konzentrationen werden ins Verhältnis gesetzt und entsprechend der nachfolgenden Formel die relative Albumin-Bindungs-Funktion der Bindungsstelle II (rABF II) ermittelt.

$$\mathrm{rABF\ II} = \frac{M_R}{M_P}\ bzw.\frac{C_{MP}}{C_{MR}}$$

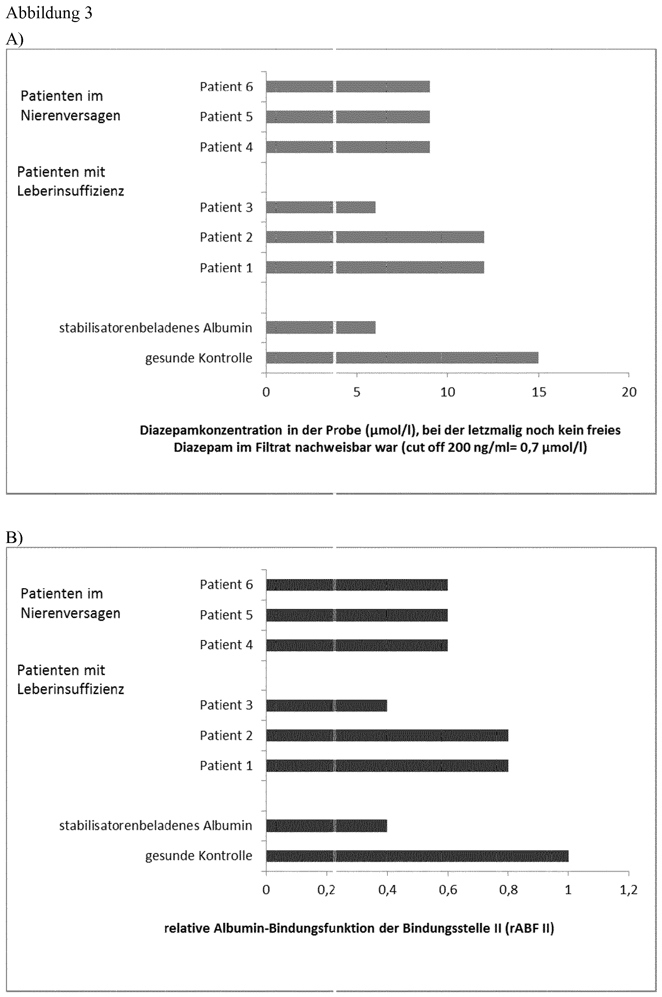

Mit: $M_R$ bzw. $M_P$ Menge des ungebundenen Markers (bei Bestimmung der Konzentrationen) oder $C_{MP}$ bzw. $C_{MR}$ Konzentrations- bzw. Verdünnungsstufe des zugegebenen Markers, bei der in der Referenz bzw. in der Probe erstmalig ungebundener Marker bzw. letztmalig KEIN ungebundener Marker im Filtrat mittels Streifentest detektiert werden kann.
Dieses wurde mit Plasma eines gesunden Freiwilligen (Referenz), einer stabilisatorenhaltigen pharmazeutischen Albuminpräparation sowie 3 Plasmaproben von Patienten mit einer chronischen Leberschädigung und 3 Patienten mit einer terminalen Niereninsuffizienz durchgeführt (siehe Abbildung 3A/B).

**Beispiel 5:** Semiquantitative Abschätzung der Diazepamkonzentration mittels Teststreifen mit unterschiedlichen Detektionsgrenzen

Eine Plasmaprobe wird mit PBS derart verdünnt, dass die Albuminkonzentration in der Probe anschließend 677 μmol/l beträgt. Zu 1,8 ml dieser Probe werden 0,2 ml einer Diazepam-Lösung mit einer Konzentration von 500 μmol/l gegeben, so dass in der Probe anschließend eine eine Albuminkonzentration von 600 μmol/l und eine Diazepamkonzentrationen von 50 μmol/l vorliegt, welches einem molaren Verhältnissen Diazepam/Albumin von 0,083 entspricht. Nach einer Inkubationszeit werden durch Zentrifugation (Centrisart Sartorius, cut off 20 000 Dalton) die ungebundenen Markermengen separiert. Nachfolgend wird die Diazepamkonzentration im Filtrat mittels (mindestens) zweier verschiedener Streifentest bestimmt (Teststreifen cut off 200 ng/ml und 300 ng/ml). Bei dem eingesetzten Streifentest erfolgt ein Signal, wenn die Diazepamkonzentration in der Flüssigkeit (Filtrat) unterhalb des angegebene cut off Wertes (200 bzw. 300 ng/ml) liegt. Somit ist eine Abschätzung der freien Diazepam-Konzentration im Filtrat möglich. In unserem Anwendungsbeispiel beträgt die freie Diazepam-Konzentration demnach weniger als 200 ng/ml, wenn in beiden eingesetzten Teststreifen ein Signal generiert wird; ist bei beiden Teststreifen kein Testsignal zu detektieren, liegt die Diazepamkonzentration im Filtrat oberhalb von 300 ng/ml.

Ist jedoch auf dem Testreifen mit dem 300 ng/ml cut-off ein Signal vorhanden, auf dem Teststreifen mit dem 200 ng/ml cut-off jedoch nicht, kann die Diazepamkonzentration im Filtrat im Bereich zwischen 200 und 300 ng/ml angegeben werden.

Durch geeignete Auswahl und Kombinationen der Teststreifen ist somit eine semiquantitative Abschätzung der Diazepamkonzentration im Filtrat möglich und eine Bestimmung der relativen Albumin-Bindungs-Funktion kann entsprechend erfolgen.

## Patentansprüche

1. Verfahren zur Bestimmung der relativen Bindungskapazität des Albumins umfassend:

     a) Bereitstellen von mindestens zwei Messlösungen einer Test-Probe und einer Referenz-Probe, wobei die Messlösungen mindestens einen Albumin-bindenden Marker M enthalten und dieser mindestens eine Albumin-bindende Marker M in mindestens einer Messlösung der Test-Probe und der Referenz-Probe die vermutlich vorhandene Bindungskapazität des Albumins übersteigt und wobei die Test-Probe eine bestimmten Menge an Albumin mit unbekannter Bindungskapazität und die Referenz-Probe die gleiche bestimmte Menge an Albumin mit einer Referenz-Bindungskapazität aufweist;
     b) Inkubieren der Messlösungen unter Bedingungen, die es erlauben, dass der mindestens eine Albumin-bindende Marker M an Albumin binden kann, sodass Komplexe aus diesem Marker M und Albumin (M:A) entstehen;
     c) Abtrennen der in Schritt b) erzeugten Komplexe (M:A);
     d) Nachweisen des Vorhandenseins oder der Menge an ungebundenem Marker M in den Lösungen nach Abtrennen des Komplexes (M:A) durch mindestens einen Teststreifen, der die Bestimmung des ungebundenen Markers erlaubt; und
     e) Bestimmen der relativen Bindungskapazität des Albumins in der Test-Probe basierend auf dem Vorhandensein oder den nachgewiesenen Mengen an ungebundenem Marker M in Schritt d),

wobei der mindestens eine Albumin-bindende Marker M ein Benzodiazepin, Tryptophan, eine Gallensäure, Dansylsarcosin, eine mittelkettigen Fettsäure, Warfarin, Furosemid, ein Sulfonylharnstoff, Dansylamid, ein Opioid, Kokain oder ein Cannabinoid ist.

2. Verfahren nach Anspruch 1, wobei der mindestens eine Albumin-bindende Marker M an die Bindungsstelle I und/oder II des Albumins bindet.

3. Verfahren nach Anspruch 2, wobei der an die Bindungsstelle I bindende Marker M Warfarin, Furosemid oder Dansylamid ist, und/oder wobei der an die Bindungsstelle II bindende Marker M Diazepam, Tryptophan, eine Gallensäure, Dansylsarcosin oder eine mittelkettige Fettsäure ist.

4. Verfahren nach einem der Ansprüche 1 bis 3, wobei der mindestens eine Albumin-bindende Marker M ein Benzodiazepin, bevorzugt Diazepam, ist.

5. Verfahren nach einem der Ansprüche 1 bis 4, wobei der mindesten eine Teststreifen eine vorbestimmte Detektionsgrenze und/oder eine vorbestimmte Toleranzgrenze für den mindestens einen Albumin-bindende Marker M aufweist.

6. Verfahren nach einem der Ansprüche 1 bis 5, wobei mehrere Teststreifen für unterschiedliche Albumin-bindende Marker M eingesetzt werden.

7. Verfahren nach Anspruch 5 oder 6, wobei die vorbestimmte Detektionsgrenze für den Albumin-bindenden Marker M bei mindestens 100ng/ml liegt und/oder eine vorbestimmte Toleranzgrenze bei etwa 200ng/ml liegt.

8. Verfahren nach einem der Ansprüche 1 bis 7, wobei die Test-Probe eine Probe aus einem Patienten mit einer Leberschädigung und/oder einer Niereninsuffizienz und/oder einer Sepsis oder eine Albuminhaltige Lösung ist.

9. Verfahren nach einem der Ansprüche 1 bis 8, wobei die Referenz-Probe eine Probe eines gesunden Probanden oder eine artifiziell hergestellte Albumin-Lösung ist.

10. Verfahren zur Bestimmung der Menge an funktionellem Albumin, umfassend:

     a) Bereitstellen einer Test-Probe mit einer bestimmten Menge an Albumin mit unbekannter Bindungskapazität und einer Referenz-Probe mit der gleichen bestimmten Menge an Albumin mit einer Referenz-Bindungskapazität;
     b) Inkubieren der Test-Probe und der Referenz Probe mit einer definierten Menge mindestens eines Albumin-bindenden Markers M unter Bedingungen, die es erlauben, dass der mindestens eine Albumin-bindende Marker M an Albumin binden kann, sodass Komplexe aus diesem Marker M und Albumin (M:A) entstehen;

c) Abtrennen der in Schritt c) erzeugten Komplexe (M:A);

d) Nachweisen des Vorhandenseins oder der Menge an ungebundenem Marker M in den Proben nach Abtrennen des Komplexes (M:A) durch einen ersten und einen zweiten Teststreifen, die die Bestimmung der Menge des ungebundenen Markers erlauben, wobei die Teststreifen unterschiedliche Toleranzgrenzen aufweisen; und

e) Bestimmen der Menge an funktionellem Albumin basierend auf dem Vorhandensein oder den nachgewiesenen Mengen des Markers M in Schritt d),

wobei der mindestens eine Albumin-bindende Marker M ein Benzodiazepin, Tryptophan, eine Gallensäure, Dansylsarcosin, eine mittelkettigen Fettsäure, Warfarin, Furosemid, ein Sulfonylharnstoff, Dansylamid, ein Opioid, Kokain oder ein Cannabinoid ist.

**11.** Verfahren nach Anspruch 10, wobei der erste Teststreifen eine vorbestimmte Toleranzgrenze von etwa 200ng/ml aufweist und/oder der zweite Teststreifen eine vorbestimmte Toleranzgrenze von etwa 300ng/ml aufweist.

**12.** Verfahren nach einem der Ansprüche 10 bis 12, wobei der mindestens eine Albumin-bindende Marker M ein Benzodiazepin, bevorzugt Diazepam, ist.

**Claims**

**1.** Method for determining the relative binding capacity of albumin, comprising:

a) providing at least two measurement solutions of a test sample and of a reference sample, wherein the measurement solutions contain at least one albumin-binding marker M and this at least one albumin-binding marker M in at least one measurement solution of the test sample and of the reference sample exceeds the presumed available binding capacity of albumin and wherein the test sample contains a defined amount of albumin of unknown binding capacity and the reference sample contains the same defined amount of albumin having a reference binding capacity;

b) incubating the measurement solutions under conditions that allow the at least one albumin-binding marker M to bind to albumin to form complexes of this marker M and albumin (M:A);

c) removing the complexes (M:A) produced in step b);

d) detecting the presence or amount of unbound marker M in the solutions after removal of the complex (M:A) by at least one test strip that allows determination of the unbound marker; and

e) determining the relative binding capacity of albumin in the test sample based on the presence or detected amounts of unbound marker M in step d),

wherein the at least one albumin-binding marker M is a benzodiazepine, tryptophan, a bile acid, dansylsarcosine, a medium-chain fatty acid, warfarin, furosemide, a sulfonylurea, dansylamide, an opioid, cocaine or a cannabinoid.

**2.** Method according to Claim 1, wherein the at least one albumin-binding marker M binds to binding site I and/or II of albumin.

**3.** Method according to Claim 2, wherein the marker M that binds to binding site I is warfarin, furosemide or dansylamide and/or wherein the marker M that binds to binding site II is diazepam, tryptophan, a bile acid, dansylsarcosine or a medium-chain fatty acid.

**4.** Method according to any of Claims 1 to 3, wherein the at least one albumin-binding marker M is a benzodiazepine, preferably diazepam.

**5.** Method according to any of Claims 1 to 4, wherein the at least one test strip has a predetermined detection limit and/or a predetermined tolerance limit for the at least one albumin-binding marker M.

**6.** Method according to any of Claims 1 to 5, wherein multiple test strips are used for different albumin-binding markers M.

**7.** Method according to Claim 5 or 6, wherein the predetermined detection limit for the albumin-binding marker M is at least 100 ng/ml and/or a predetermined tolerance limit is about 200 ng/ml.

**8.** Method according to any of Claims 1 to 7, wherein the test sample is a sample from a patient with liver damage and/or renal insufficiency and/or sepsis or an albumin-containing solution.

**9.** Method according to any of Claims 1 to 8, wherein the reference sample is a sample from a healthy subject or a synthetically produced albumin solution.

**10.** Method for determining the amount of functional albumin, comprising:

a) providing a test sample containing a defined amount of albumin of unknown binding capacity and a reference sample containing the same defined amount of albumin having a reference binding capacity;

b) incubating the test sample and reference sample with a defined amount of at least one albumin-binding marker M under conditions that allow the at least one albumin-binding marker M to bind to albumin to form complexes of this marker M and albumin (M:A);

c) removing the complexes (M:A) formed in step c);

d) detecting the presence or amount of unbound marker M in the samples after removal of the complex (M:A) through a first and a second test strip that allow determination of the amount of unbound marker, with the test strips having different tolerance limits; and

e) determining the amount of functional albumin based on the presence or detected amounts of marker M in step d),

wherein the at least one albumin-binding marker M is a benzodiazepine, tryptophan, a bile acid, dansylsarcosine, a medium-chain fatty acid, warfarin, furosemide, a sulfonylurea, dansylamide, an opioid, cocaine or a cannabinoid.

**11.** Method according to Claim 10, wherein the first test strip has a predetermined tolerance limit of about 200 ng/ml and/or the second test strip has a predetermined tolerance limit of about 300 ng/ml.

**12.** Method according to any of Claims 10 to 12, wherein the at least one albumin-binding marker M is a benzodiazepine, preferably diazepam.

## Revendications

**1.** Procédé de détermination de la capacité relative de liaison de l'albumine, comprenant :

a) la préparation d'au moins deux solutions de mesure d'un échantillon à tester et d'un échantillon de référence, les solutions de mesure contenant au moins un marqueur M de liaison d'albumine et ledit au moins un marqueur de liaison d'albumine M dépassant dans au moins une solution de mesure de l'échantillon à tester et de l'échantillon de référence la capacité de liaison vraisemblablement existante de l'albumine et l'échantillon à tester comportant une quantité déterminée en albumine avec une capacité de liaison inconnue et l'échantillon de référence comportant la même quantité déterminée d'albumine avec une capacité de liaison de référence ;

b) l'incubation des solutions de mesure dans des conditions qui permettent que l'au moins un marqueur de liaison d'albumine M puisse se lier à l'albumine de manière à produire des complexes à partir dudit marqueur M et de l'albumine (M:A) ;

c) la séparation des complexes (M:A) produits à l'étape b) ;

d) la démonstration de la présence ou de la quantité du marqueur M non lié dans les solutions après la séparation du complexe (M:A) par au moins une bandelette de test, qui permet de déterminer le marqueur non lié ; et

e) la détermination de la capacité relative de liaison de l'albumine dans l'échantillon à tester sur la base de la présence ou des quantités démontrées en marqueur M non lié à l'étape d),

l'au moins un marqueur de liaison d'albumine M étant une benzodiazépine, un tryptophane, un acide biliaire, de la dansylsarcosine, un acide gras à chaîne moyenne, de la warfarine, du furosémide, une sulfonylurée, du dansylamide, un opioïde, de la cocaïne ou un cannabinoïde.

**2.** Procédé selon la revendication 1, l'au moins un marqueur M de liaison d'albumine se liant à l'emplacement de liaison I et/ou II de l'albumine.

**3.** Procédé selon la revendication 2, le marqueur M de liaison à l'emplacement de liaison I étant de la warfarine, du furosémide ou du dansylamide, et/ou l'au moins un marqueur M se liant à l'emplacement de liaison II étant du diazépam, du tryptophane, un acide biliaire, de la dansylsarcosine ou un acide gras à chaîne moyenne.

**4.** Procédé selon l'une des revendications 1 à 3, l'au moins un marqueur M de liaison d'albumine étant une benzodiazépine, de manière préférée du diazépam.

**5.** Procédé selon l'une des revendications 1 à 4, l'au moins une bandelette de test comportant une limite de détection prédéterminée et/ou une limite de tolérance prédéterminée pour l'au moins un marqueur M de liaison d'albumine.

**6.** Procédé selon l'une des revendications 1 à 5, plusieurs bandelettes de test pour différents marqueurs M de liaison d'albumine étant employées.

**7.** Procédé selon la revendication 5 ou 6, la limite de détection prédéterminée pour le marqueur M de liaison d'albumine étant de l'ordre d'au moins 100 ng/ml et/ou une limite de tolérance prédéterminée étant de l'ordre d'environ 200 ng/ml.

**8.** Procédé selon l'une des revendications 1 à 7, l'échantillon à tester étant un échantillon provenant d'un patient avec une lésion hépatique et/ou une insuffisance rénale et/ou une septicémie ou une solution contenant de l'albumine.

**9.** Procédé selon l'une des revendications 1 à 8, l'échantillon de référence étant un échantillon d'un sujet sain ou une solution d'albumine produite de manière artificielle.

**10.** Procédé de détermination de la quantité d'albumine fonctionnelle, comprenant :

a) la préparation d'un échantillon à tester avec une quantité déterminée d'albumine avec une capacité de liaison inconnue et d'un échantillon de référence avec la même quantité déterminée en albumine avec une capacité de liaison de référence ;

b) l'incubation de l'échantillon à tester et de l'échantillon de référence avec une quantité définie d'au moins un marqueur M de liaison d'albumine dans des conditions qui permettent que l'au moins un marqueur M de liaison d'albumine puisse se lier à l'albumine de manière à produire des complexes à partir dudit marqueur M et de l'albumine (M:A) ;

c) la séparation des complexes (M:A) produits à l'étape c) ;

d) la démonstration de la présence ou de la quantité d'un marqueur M non lié dans les échantillons après séparation du complexe (M:A) par une première et une deuxième bandelette de test, qui permettent de déterminer la quantité du marqueur non lié, les bandelettes de test présentant des limites de tolérance différentes ; et

e) la détermination de la quantité d'albumine fonctionnelle sur la base de la présence ou des quantités démontrées du marqueur M à l'étape d),

l'au moins un marqueur de liaison d'albumine M étant une benzodiazépine, un tryptophane, un acide biliaire, de la dansylsarcosine, un acide gras à chaîne moyenne, de la warfarine, du furosémide, une sulfonylurée, du dansylamide, un opioïde, de la cocaïne ou un cannabinoïde.

**11.** Procédé selon la revendication 10, la première bandelette de test présentant une limite de tolérance prédéterminée d'environ 200 ng/ml et/ou la deuxième bandelette de test présentant une limite de tolérance prédéterminée d'environ 300 ng/ml.

**12.** Procédé selon l'une des revendications 10 à 12, l'au moins un marqueur M de liaison d'albumine étant une benzodiazépine, de manière préférée du diazépam.

Abbildung 1

Abbildung 2

Abbildung 3

A)

Diazepamkonzentration in der Probe (µmol/l), bei der letzmalig noch kein freies Diazepam im Filtrat nachweisbar war (cut off 200 ng/ml= 0,7 µmol/l)

B)

relative Albumin-Bindungsfunktion der Bindungsstelle II (rABF II)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1315973 B1

**In der Beschreibung aufgeführte Nicht-Patentliteratur**

- **KRAGH**. Practical aspects of the ligand-binding and enzymatic properties of human serum albumin.. *Biol Pharm Bull*, June 2002, vol. 25 (6), 695-704
- **SUDLOW G**. The characterization of two specific drug binding sites on human serum albumin. *Mol Pharmacol*, 1975, vol. 11 (6), 824-832
- **LEE P** ; **WU X.** Review: modifications of human serum albumin and their binding effect.. *Curr Pharm Des*, 2015, vol. 21 (14), 1862-1865
- **FASSANO M. et al.** The extraordinary ligand binding properties of human serum albumin.. *IUBMB Life*, December 2005, vol. 57 (12), 787-96
- **SEN et al.** Emerging indications for albumin dialysis.. *Am J Gastroenterol*, February 2005, vol. 100 (2), 468-475
- **HUGHES RD.** Review of methods to remove protein-bound substances in liver failure.. *Int J Artif Organs*, October 2002, vol. 25 (10), 911-917
- **HEEMANN et al.** Albumin dialysis in cirrhosis with superimposed acute liver injury: A prospective, controlled study.. *Hepatology*, October 2002, vol. 36 (4), 949-958
- **LIABEUF et al.** Protein-bound uremic toxins: new insight from clinical studies. *Toxins (Basel)*, July 2011, vol. 3 (7), 911-919
- **DOU et al.** The uremic solutes p-cresol and indoxyl sulfate inhibit endothelial proliferation and wound repair.. *Kidney Int*, February 2004, vol. 65 (2), 442-451
- **KLAMMT**. Albumin-binding function is reduced in patients with decompensated cirrhosis and correlates inversely with severity of liver disease assessed by model for end-stage liver disease.. *Eur J Gastroenterol Hepatol*, March 2007, vol. 19 (3), 257-263
- **KLAMMT et al.** Albumin-binding capacity (ABiC) is reduced in patients with chronic kidney disease along with an accumulation of protein-bound uraemic toxins.. *Nephrol Dial Transplant*, 15 November 2011, vol. 27 (6), 2377-2383
- **KLAMMT et al.** Improvement of impaired albumin binding capacity in acute-on-chronic liver failure by albumin dialysis.. *Liver Transpl*, 28 August 2008, vol. 14 (9), 1333-1339
- **HINZ et al.** Albumin function is reduced in severe sepsis.. *Infection*, 2011, vol. 39, S118
- **BLENCOWE et al.** *J. Analytical Tox*, 2011, vol. 35, 349-356
- **SUDLOW G**. The characterization of two specific drug binding sites on human serum albumin.. *Mol Pharmacol*, 1975, vol. 11 (6), 824-832
- **PETERS T.** All about albumin : biochemistry, genetics, and medical applications.. Academic Press, 1996
- **GHUMAN J et al.** Structural basis of the drug-binding specificity of human serum albumin.. *J Mol Biol*, 2005, vol. 353, 38-52